# EUROPÄISCHE PATENTSCHRIFT

(11) **EP 2 758 803 B1**
(45) Veröffentlichungstag und Bekanntmachung des Hinweises auf die Patenterteilung: **16.09.2020**
(21) Anmeldenummer: 11761331.5
(22) Anmeldetag: 20.09.2011
(51) Int. Cl.: G01S 5/02, G01S 5/00, G06K 9/00, G09B 19/00, A61B 5/11, G16H 20/30

(54) **SYSTEM UND VERFAHREN ZUR UNTERSTÜTZUNG EINER BEWEGUNGSÜBUNG**
SYSTEM AND METHOD FOR SUPPORTING AN EXERCISE MOVEMENT
SYSTÈME ET PROCÉDÉ D'AIDE À UN EXERCICE DE MOUVEMENT

(43) Veröffentlichungstag der Anmeldung: 30.07.2014
(73) Patentinhaber: Fraunhofer Gesellschaft zur Förderung der angewandten Forschung e.V., 80686 München (DE)
(72) Erfinder: LOCHMANN, Matthias, 91077 Neunkirchen am Brand (DE)
(74) Vertreter: Patentship Patentanwaltsgesellschaft mbH
(86) Internationale Anmeldenummer: PCT/EP2011/066263
(87) Internationale Veröffentlichungsnummer: WO 2013/041123

(56) Entgegenhaltungen:
- US-A1- 2004 147 329
- US-A1- 2009 102 746
- US-B1- 7 018 211
- US-B1- 7 302 288
- KNEISSLER M ET AL: "Concept and clinical evaluation of navigated control in spine surgery", ADVANCED INTELLIGENT MECHATRONICS, 2003. AIM 2003. PROCEEDINGS. 2003 I EEE/ASME INTERNATIONAL CONFERENCE ON JULY 20 - JULY 24, 2003, PISCATAWAY, NJ, USA,IEEE, Bd. 2, 20. Juli 2003 (2003-07-20), Seiten 1084-1089, XP010654629, ISBN: 978-0-7803-7759-2

## Beschreibung

Die vorliegende Erfindung betrifft das Gebiet der Bewegungsübungen im Sport- und Rehabilitationsbereich.

Die menschliche Bewegung ist ein komplexer biomechanischer Ablauf. So muss beispielsweise ein Sportler, welcher einen Sportgegenstand, wie etwa einen Ball oder ein Wurfgerät, verwendet, im Rahmen eines Trainings diejenigen Bewegungsabläufe einüben, welche zu einem optimalen Sportergebnis führen. Wesentlich komplizierter ist die Lage bei so genannten Mannschaftssportarten, wo beispielsweise eine technisch-taktische Interaktion von Sportlern untereinander sowie mit einem Spiel- bzw. Wurfgerät während eines Trainings eingeübt werden muss.

Zur Trainingsanalyse sind technische Vorrichtungen bekannt, welche beispielsweise eine Videoanalyse eines bereits erfolgten Spiels ermöglichen. Eine derartige Videoanalyse ist hilfreich, um eine Aussage über die Bewegungen der einzelnen Spieler bezüglich einander oder bezüglich des Balls treffen zu können.

Ein Nachteil derartiger Vorrichtungen besteht darin, dass sie nicht zur Unterstützung der Bewegungsausübung in Echtzeit eingesetzt werden können. Aus diesem Grund sind derartige Vorrichtungen auch für den Rehabilitationsbereich, wo es besonders wichtig ist, einen Rehabilitationspatienten bereits während der Unterstützung einer Bewegungsübung zu unterstützen, nicht geeignet.

Die Druckschrift US 2009/102746 A1 offenbart ein System zum Visualisieren oder Beobachten einer körperlichen Aktivität. Das System umfasst eine Videokamera, welche einen Nutzer bei einer körperlichen Übung aufzeichnet. Ferner ist eine Anzeigeeinrichtung vorgesehen, die von dem Nutzer getragen wird und mit einem Computer verbunden ist. Die Anzeigeeinrichtung zeigt das aufgenommene Bild des Nutzers an.

Die Druckschrift US 7 302 288 B1 offenbart ein Werkzeug zur Verwendung in einem medizinischen Operationsnavigationssystem, welches eine Mehrzahl von LEDs umfasst, welche an einem Ende des Werkzeugs angebracht sind. Die LEDs zeigen eine Richtung an, in welche das Werkzeug bewegt werden sollte, um eine gewünschte Position zu erreichen.

Die Druckschrift Kneissler M. et al., "Concept and Clinical Evaluation of Navigated Control in Spine Surgery", IEEE/ASME International Conference on Advanced Intelligent Mechatronics, 2003, offenbart ein Operationssystem, welches eine relative Position eines chirurgischen Instruments bezüglich einer Patientenanatomie bestimmen kann.

Die Druckschrift US 7 018 211 B1 offenbart ein System, welches es ermöglichen soll, dass eine sich bewegende Person ihre Körperbewegungen überprüfen kann. Mittels einer Kamera wird ein Video der Person aufgezeichnet, welches dann auf einem Monitor angezeigt wird. Ferner sind Marker vorgesehen, welche ebenfalls auf dem Monitor dargestellt werden.

Die Druckschrift US 2004/0147329 A1 offenbart ein persönliches Golf-Assistenzsystem mit einer Software, welche auf einem PDA ausgeführt wird, welcher mit einem GPS-Empfänger verbunden ist, und welcher einem Nutzer erlaubt, geophysikalische Golf-Daten zu überwachen und/oder elektronisch zu erfassen.

Es ist eine Aufgabe der vorliegenden Erfindung, ein Konzept zur Unterstützung einer Bewegungsübung in Echtzeit zu schaffen.

Diese Aufgabe wird durch die Merkmale der unabhängigen Ansprüche gelöst. Vorteilhafte Weiterbildungsformen sind Gegenstand der abhängigen Ansprüche, der Beschreibung sowie der Figuren.

Die vorliegende Erfindung basiert auf der Erkenntnis, dass die obige Aufgabe durch eine Bestimmung und eine Anzeige einer Soll-Position des Objektes realisiert werden kann, sofern sich die Soll-Position von der Ist-Position des Objektes unterscheidet. Auf diese Weise kann beispielsweise einem Torwart, der gemäß einer Ausführungsform das Objekt darstellt, eine Verbindungslinie zwischen der Tormitte und dem Ball als Torwart-Soll-Position bereits während eines Trainings in Echtzeit angezeigt werden. Auf diese Weise kann der Torwart seine Bewegung bezüglich der Geometrie des Spielfeldes schneller koordinieren. Ist das Objekt der Ball, so kann beispielsweise einem Fußballspieler in Echtzeit in Abhängigkeit von der geometrischen Position eines weiteren Spielers eine Soll-Position des Balls in Abhängigkeit von einem Spielgeschehen angezeigt werden.

Dadurch kann die Qualität des technisch-taktischen Trainings in Echtzeit optimiert werden. Insbesondere kann ein definiertes Zielverhalten auf der Grundlage von Normwerten auf der Basis von empirisch-statistischen Modellen und/oder auf der Basis von deterministischen Modellen und/oder gemischten Modellansätzen via permanentem Echtzeitfeedback in wesentlich kürzeren Lern- und Trainingsintervallen ermöglicht werden als dies mit bisherigen Verfahren möglich gewesen ist. Die Erfindung kann insbesondere dazu beitragen, komplexe Abläufe, beispielsweise die technisch-taktische Interaktion von Sportlern untereinander oder mit einem Spiel- bzw. Wurfgerät während eines sportlichen Trainings im Detail und in Echtzeit zu analysieren und auf der Basis von einem Soll-Istwert Vergleichen ein Training oder Spiel in Echtzeit zu steuern.

Soll eine Bewegungsübung beispielsweise mit einem Kniegelenk als Objekt während einer Rehabilitation durchgeführt werden, so kann die Region des Knies eines Rehabilitationspatienten mit einem Positionssensor versehen werden, welcher die Ist-Position des Knies anzeigt. Ist das Knie beispielsweise zu einem definierten Zeitpunkt einer Übung in einer nicht erwünschten Position , so kann dem Rehabilitationspatienten ein Hinweis angezeigt werden, dass die Soll-Position noch nicht erreicht wurde oder in welche Richtung das Knie zu bewegen ist, um die Soll-Position zu erreichen. Auf diese Weise können rasche Rehabilitationsfortschritte erzielt werden.

Das Objekt kann ein Spielgegenstand, wie beispielsweise ein Fußball oder ein Tennisschläger, der Spieler selbst, ein Körperbestandteil des Spielers, wie beispielsweise dessen Kopf oder dessen Fuß, sein. Gemäß einigen Ausführungsformen kann das Objekt ein Rehabilitationspatient bzw. ein Körperbestandteil des Rehabilitationspatienten, wie etwa ein Kniegelenk oder ein Ellbogengelenk, sein. Das Objekt kann jedoch auch ein Rehabilitationsobjekt sein, wie etwa ein Gymnastikball.

Gemäß einem ersten Aspekt betrifft die Erfindung ein System zur Unterstützung einer Bewegungsübung mit einem Objekt, mit einer Erfassungseinrichtung zum Erfassen einer Ist-Position des Objektes, einer Bestimmungseinrichtung zum Bestimmen einer Soll-Position des Objektes und einer Anzeigeeinrichtung zum Anzeigen eines Hinweises auf die Soll-Position, wenn sich die Ist-Position von der Soll-Position unterscheidet.

Der Hinweis auf die Soll-Position kann eine Anzeige der Soll-Position selbst oder eine Anzeige, dass die Soll-Position noch nicht erreicht wurde, oder eine Anzeige einer Richtung der Soll-Position umfassen. Zur Feststellung, ob sich die Ist-Position von der Soll-Position unterscheidet, kann die Bestimmungseinrichtung die Ist-Position mit der Soll-Position vergleichen. Hierbei können die Ist-Position und die Soll-Position in der Gestalt von digitalen Positionsdaten vorliegen.

Gemäß einer Ausführungsform ist das Objekt ausgebildet, ein Positionssignal, insbesondere ein Global Positioning Signal, auszusenden. Die Erfassungseinrichtung ist ausgebildet, das Positionssignal zu empfangen und die Ist-Position des Objektes auf der Basis des empfangenen Positionssignals zu bestimmen. Zum Empfang des Positionssignals kann die Erfassungseinrichtung eine Empfangsantenne umfassen, welche das Positionssignal empfängt. Die Erfassungseinrichtung kann ferner ausgebildet sein, das empfangene Positionssignal zu verarbeiten, um die Ist-Position des Objekts zu erfassen.

Gemäß einer Ausführungsform ist die Erfassungseinrichtung ausgebildet, ein an dem Objekt reflektiertes Reflexionssignal, insbesondere ein Radarsignal oder ein Lasersignal, zu empfangen und auf der Basis des reflektierten Reflexionssignals die Ist-Position des Objektes zu bestimmen. Das Objekt kann hierzu beispielsweise eine reflektierende Oberfläche aufweisen, welche metallisch sein kann, um ein Radarsignal zu reflektieren.

Gemäß einer Ausführungsform ist die Erfassungseinrichtung ausgebildet, ein Sendesignal, insbesondere ein Radarsignal oder ein Lasersignal, auszusenden, um das Reflexionssignal zu erzeugen. Hierzu kann die Erfassungseinrichtung einen Sender, insbesondere eine Sendeantenne, umfassen, welche ein gerichtetes oder ein nicht gerichtetes Sendesignal aussendet, das an dem Objekt reflektiert wird, wodurch das Reflexionssignal erzeugt wird.

Gemäß einer Ausführungsform ist die Bestimmungseinrichtung ausgebildet, eine vorbestimmte Position des Objektes, insbesondere eine vorgebbare Position des Objektes, als die Soll-Position zu bestimmen. Die vorbestimmte Position des Objektes kann beispielsweise aus einer Mehrzahl von möglichen Soll-Positionen ausgewählt werden. Diese Auswahl kann zufällig oder deterministisch sein. Die vorbestimmte, deterministische Position kann beispielsweise anhand einer Regel vorgegeben werden.

Gemäß einer Ausführungsform ist das Objekt ein Ball, und die Bestimmungseinrichtung ist ausgebildet, einen Sensorbereich einer Sensorwand als die Soll-Position zu bestimmen, insbesondere auszuwählen.

Gemäß einer Ausführungsform ist die Sensorwand ein Element des Systems. Gemäß einer anderen Ausführungsform ist die Sensorwand kein Element des Systems. Die Sensorwand kann zumindest einen Sensor, beispielsweise einen Drucksensor, zur Detektion des Balls aufweisen. Die Sensorwand kann gemäß einer Ausführungsform jedoch derart gebildet sein, dass das Eintreffen des Balls durch beispielsweise eine Einwölbung oder Auswölbung der Sensorwand beispielsweise optisch detektierbar ist.

Auf diese Weise kann beispielsweise einem Fußballspieler als Soll-Position des Balls ein Bereich der Sensorwand angezeigt werden, welcher ein Tor darstellt. Der Fußballspieler kann auf diese Weise rasch unterschiedliche Ballschießübungen ausführen.

Gemäß einer Ausführungsform kann das System einen oder mehrere Ballgeber umfassen, welcher einen Ball als das zu dem Sensorbereich hin zu befördernde Objekt abgibt. Der Ballgeber kann ausgebildet sein, den Ball in eine vorbestimmte Richtung, beispielsweise zum Spieler hin, abzugeben. Die Anzeigeeinrichtung kann durch die Sensorwand gebildet sein, welche den Sensorbereich als die Soll-Position anzeigt.

Gemäß einer Ausführungsform ist die Bestimmungseinrichtung ausgebildet, die Soll-Position in Abhängigkeit von der Ist-Position des Objektes zu bestimmen. Auf diese Weise wird eine dynamische Bestimmung der Soll-Position in Abhängigkeit von beispielsweise einem Spielgeschehen auf einem Spielfeld ermöglicht.

Gemäß einer Ausführungsform ist die Bestimmungseinrichtung ausgebildet, die Soll-Position in Abhängigkeit von der Ist-Position des Objektes und einer geometrischen Charakteristik eines Bereichs, insbesondere eines Spielfeldes, innerhalb dessen das Objekt bewegbar ist, oder die Soll-Position in Abhängigkeit von der Ist-Position des Objektes bezüglich einer geometrischen Charakteristik des Spielfeldes, insbesondere einer Tormitte, zu bestimmen. So kann beispielsweise die Soll-Position als eine Verbindungslinie zwischen Tormitte und Ball in Abhängigkeit von einer Ballposition bestimmt werden.

Gemäß einer Ausführungsform ist die Bestimmungseinrichtung ausgebildet, die Soll-Position anhand einer Regel, insbesondere anhand einer biomechanischen Regel, zu bestimmen. Handelt es sich beispielsweise bei dem Objekt um einen zu bewegenden Körperbestandteil eines Rehabilitationspatienten, so kann bei der Bestimmung der Soll-Position die biomechanische Charakteristik des menschlichen Körpers berücksichtigt werden. Die biomechanische Regel kann jedoch auch das Alter des Rehabilitationspatienten oder dessen grundsätzliche Beweglichkeit umfassen. Auf diese Weise kann vermieden werden, dass unerreichbare Soll-Positionen bestimmt und angezeigt werden.
Gemäß einer Ausführungsform ist die Bestimmungseinrichtung ausgebildet, die Soll-Position als eine Abfolge sukzessiver Hilfs-Positionen zu bestimmen. Durch die sukzessive Abfolge der Hilfspositionen wird das schrittweise Erreichen einer endgültigen Soll-Position vereinfacht, da beispielsweise kleinere Bewegungsschritte geübt werden können.

Gemäß einer Ausführungsform ist die Erfassungseinrichtung ausgebildet, eine weitere Ist-Positionen von einem weiteren Objekt zu bestimmen, und die Bestimmungseinrichtung ist ausgebildet, die Soll-Position des Objektes in Abhängigkeit von der weiteren Ist-Position, insbesondere relativ zu der weiteren Ist-Position des weiteren Objektes zu bestimmen. Handelt es sich bei dem Objekt um einen Spieler, so kann das weitere Objekt ein anderer Spieler sein, wodurch die Bewegung des Spielers bezüglich des weiteren Spielers geübt werden. Handelt es sich bei dem Objekt beispielsweise um einen Ball, so kann das weitere Objekt ein Spieler, beispielsweise ein Torwart, sein und umgekehrt. Auf diese Weise kann die Soll-Position des Balles oder des Spielers in Abhängigkeit von der weiteren Soll-Position beispielsweise dynamisch bestimmt und angezeigt werden.

Gemäß einer Ausführungsform ist die Bestimmungseinrichtung ausgebildet, die Soll-Position anhand einer vorgegeben Regel, welche die Soll-Position mit einer Ist-Position verknüpft, zu bestimmen. Die Regel kann beispielsweise die Soll-Position eines Spielers bezüglich der weiteren Ist-Position eines weiteren Spielers verknüpfen.

Gemäß einer Ausführungsform sind das Objekt und das weitere Objekt Skier. Auf diese Weise kann beispielsweise eine Stellung der Skier bezüglich einander, insbesondere beim Skisprung, angezeigt werden.

Gemäß einer Ausführungsform ist die Erfassungseinrichtung ausgebildet, eine Mehrzahl von weiteren Ist-Positionen von einer Mehrzahl von weiteren Objekten zu bestimmen, und die Bestimmungseinrichtung ist ausgebildet, die Soll-Position des Objektes in Abhängigkeit von der Mehrzahl der weiteren Ist-Positionen zu bestimmen. Die Mehrzahl der weiteren Ist-Positionen kann beispielsweise durch Ist-Positionen von Spielern einer Spielmannschaft bestimmt werden. Auf diese Weise kann die Soll-Position eines einzelnen Spielers bezüglich der Spielmannschaft bestimmt und/oder angezeigt werden.

Gemäß einer Ausführungsform ist die Bestimmungseinrichtung ausgebildet, einen Schwerpunkt, insbesondere einen geometrischen oder gewichteten Schwerpunkt, der weiteren Ist-Positionen zu bestimmen, und die Soll-Position in Abhängigkeit von dem Schwerpunkt, insbesondere relativ zu dem Schwerpunkt, zu bestimmen. Der geometrische Schwerpunkt kann beispielsweise anhand jedes an sich bekannten Algorithmus bestimmt werden, welcher eine Bestimmung eines geometrischen Schwerpunktes ermöglicht. Der gewichtete Schwerpunkt kann beispielsweise einen geometrischen Schwerpunkt von gemäß einer Ausführungsform gewichteten Ist-Positionen der weiteren Objekte sein. Bei der Wichtung kann beispielsweise die Bedeutung eines Spielers, beispielsweise eines Torwarts, für eine bestimmte Spielsituation zum Ausdruck gebracht werden.

Gemäß einer Ausführungsform ist die Bestimmungseinrichtung ausgebildet, die Soll-Position in Abhängigkeit von dem geometrischen Schwerpunkt anhand einer vorgegebenen Regel, welche Soll-Positionen mit geometrischen Schwerpunkten verknüpft, zu bestimmen. Diese Regel kann beispielsweise anhand von Erfahrungswerten erstellt werden.

Gemäß einer Ausführungsform ist die Anzeigeeinrichtung ausgebildet, als Hinweis auf die Soll-Position die Soll-Position selbst oder einen Hinweis auf die Lage der Soll-Position bezüglich einer Lage des Objektes, insbesondere auf eine Richtung zu der Soll-Position, oder als einen Hinweis auf eine Differenz zwischen der Ist-Position und der Soll-Position anzuzeigen. Die Differenz kann beispielsweise durch ein akustisches Signal angezeigt werden, beispielsweise eine Schwebung oder ein in der Frequenz veränderliches Signal, wobei die Schwebungsfrequenz oder die Signalfrequenz unmittelbar von der Differenz abhängen.

Gemäß einer Ausführungsform ist die Anzeigeeinrichtung ausgebildet, den Hinweis auf die Soll-Position akustisch, optisch, akusto-optisch, taktil, insbesondere mittels einer Vibration oder eines Drucks, anzuzeigen. Die Anzeigevorrichtung kann hierzu durch beispielsweise einen Benutzer tragbar sein, um ein beispielsweise taktiles Signal zu erzeugen, das dem Benutzer, beispielsweise Spieler, den Hinweis auf die Soll-Position anzeigt. Die Anzeigeeinrichtung kann jedoch einen Bildschirm umfassen oder ausgebildet sein, den Hinweis auf die Projektion auf eine Projektionsfläche, beispielsweise auf ein Spielfeld oder auf eine Visierscheibe eines Helmvisiers, zu projizieren.

Gemäß einer Ausführungsform ist das Objekt ein Spielball, insbesondere ein Fußball oder ein Tischtennisball oder ein Tennisball oder ein Rugbyball, oder ein Puck, die Anzeigeeinrichtung umfasst eine Sensorwand zum Detektieren eines auf die Sensorwand eintreffenden Objektes, und die Anzeigeeinrichtung ist ausgebildet, einen Bereich der Sensorwand als Hinweis auf die Soll-Position durch visuelles Hervorheben, insbesondere durch Leuchten oder Beleuchten des Bereichs, oder durch akustisches Hervorheben anzuzeigen. Die Sensorwand kann beispielsweise Merkmale der vorgenannten Sensorwand aufweisen oder der vorgenannten Sensorwand entsprechen.

Gemäß einer Ausführungsform umfasst die Anzeigeeinrichtung ein Visier oder eine Projektionseinrichtung, insbesondere eine Head-Up-Projektionseinrichtung, und die Anzeigeeinrichtung ist ausgebildet, den Hinweis auf die Soll-Position auf dem Visier oder durch eine Projektion auf einen Projektionsbereich, insbesondere auf eine Visierscheibe oder Windschutzscheibe, mittels der Projektionseinrichtung anzuzeigen. Die Anzeigeeinrichtung kann beispielsweise mittels der Head-Up-Projektionseinrichtung den Hinweis auf die Soll-Position auf eine Windschutzscheibe eines Fahrzeugs oder auf eine Visierscheibe eines Helmvisiers projizieren.

Gemäß einer Ausführungsform ist das Visier ein Visier eines Skihelmes, und die Anzeigeeinrichtung ist ausgebildet, den Hinweis auf die Soll-Position bezüglich der Ist-Position oder bezüglich zumindest eines der Skier bezüglich des anderen Skis auf dem Visier anzuzeigen, insbesondere als Ski-Soll-Stellung anzuzeigen. Auf diese Weise kann einem Skispringer während eines Skiflugs die Soll-Position der Skier bezüglich einander unmittelbar angezeigt werden. Hierzu können die Skier beispielsweise mit einem Sender versehen sein, welcher Positionssignale aussendet. Die Bestimmungseinrichtung kann hierbei in dem Skihelm angeordnet sein und diese Signale empfangen.

Auf diese Weise kann nicht nur die Stellung bzw. Position der Skier zueinander, sondern auch die Ist-Position der Skier im Vergleich zu einer Soll-Position, die beispielsweise vorgegeben ist oder durch ein biomechanisches Modell bestimmt ist, angezeigt werden. Bei der Bestimmung der Soll-Position können beispielsweise die aktuelle Thermik, der Seitenwind, die Luftdichte oder die Fluggeschwindigkeit, insbesondere anhand einer vorgegebenen Regel, welche die vorgenannten Größen mit Soll-Positionen verknüpft, berücksichtigt werden.

Gemäß einer Ausführungsform ist das Objekt ein Fahrzeug, insbesondere ein Schlitten oder ein Bob oder ein Kraftfahrzeug, und die Anzeigeeinrichtung ist ausgebildet, den Hinweis auf die Soll-Position als eine Fahrzeugideallinie auf einem Visier eines Helmes oder Projektion auf eine Windschutzscheibe anzuzeigen. Die Fahrzeugideallinie ist gemäß einer Ausführungsform jene Fahrtlinie, die für die aktuelle Fahrsituation die geringste Rundenzeit ermöglichen würde.

Gemäß einer Ausführungsform ist das Objekt zumindest ein Fuß eines Benutzers, insbesondere eines Golfspielers, und die Anzeigeeinrichtung ist ausgebildet, den Hinweis auf die Soll-Position zumindest eines der Füße auf eine Standfläche, auf welcher der Benutzer positionierbar ist, anzuzeigen, insbesondere zu projizieren. Der Hinweis kann beispielsweise durch eine Projektion auf die Standfläche angezeigt werden. Gemäß einer Ausführungsform kann die Standfläche beispielsweise mit Leuchtelementen, insbesondere mit Leuchtdioden, versehen sein, welche beispielsweise die gewünschte Fußstellung als Soll-Position anzeigt.

Gemäß einer Ausführungsform ist die Anzeigeeinrichtung ausgebildet, den Hinweis auf die Soll-Position auf einem elektronischen Display anzuzeigen. Die Bestimmungseinrichtung kann ausgebildet sein, das Display geeignet anzusteuern.

Gemäß einer Ausführungsform ist die Anzeigeeinrichtung ausgebildet, das Display eines Smartphones zum Anzeigen des Hinweises auf die Soll-Position anzusteuern.

Gemäß einer Ausführungsform können sowohl die Erfassungseinrichtung und/oder die Bestimmungseinrichtung und/oder die Anzeigeeinrichtung auf einem derartigen Smartphone beispielsweise in Software mittels eines Applikationsprogramms realisiert werden.

Gemäß einer Ausführungsform ist die Anzeigeeinrichtung ausgebildet, den Hinweis auf die Soll-Position auf ein Spielfeld mittels eines Lichts zu projizieren, insbesondere mittels einer Laser-Projektion oder einer LED-Projektion zu projizieren.

Gemäß einer Ausführungsform ist die Bestimmungseinrichtung ausgebildet, die Soll-Position in Abhängigkeit von einem Körperparameter, insbesondere Herzfrequenz, Herzfrequenzvariabilität, Atemfrequenz, Körpertemperatur, Blutwertparameter wie Zuckerkonzentration oder Sauerstoffkonzentration, zu bestimmen. Die Körperparameter können beispielsweise mittels berührungsloser Sensoren ermittelt und zum Beispiel an die Erfassungseinrichtung ausgesendet werden.

Gemäß einer Ausführungsform ist das Objekt ein Spielgerät, insbesondere ein Schläger wie Tennisschläger, Golfschläger, Tischtennisschläger, ein Fechtgerät, ein Gewicht, Ball, Puck, Curl, Boot, insbesondere Kanu, Paddel, ein Wurfspielgerät, insbesondere Diskus oder Hammer, Rad, Radelement wie Lenker, Bogen, Bogenpfeil, Schiessgerät, Helm, Schlittschuh, Bekleidungsstück, Ski, Skibindung, Snowboard, Fahrzeug wie Schlitten oder Bob oder Kraftfahrzeug, oder Schuh.

Gemäß einer Ausführungsform ist das Objekt ein Benutzer, insbesondere ein Spieler, oder Körperbestandteil des Benutzers, insbesondere Kopf, Stirn, Hinterkopf, Nasion, Inion, eine präaurikuläre Stelle, HWS, BWS, LWS, Brustbein, Schulterhöhe, Gelenk wie Ellenbogengelenk oder Handgelenk oder Kniegelenk oder oberes Sprunggelenk, Spina Iliaca anterior superior, Trochanter major, Fußspitze, Ferse oder Bauchnabel.

Gemäß einer Ausführungsform ist das Objekt der Kopf eines Benutzers, wobei die Erfassungseinrichtung ausgebildet ist, eine Mehrzahl von Positionssignalen zu erfassen, welche auf eine Kopfstellung hinweisen, wobei die Bestimmungseinrichtung ausgebildet ist, eine Transversalebene des Kopfes als die Ist-Position des Kopfes zu bestimmen, und wobei die Anzeigeeinrichtung ausgebildet ist, einen Hinweis auf die in der Transversalebene liegende Soll-Position, insbesondere akustisch oder optisch oder taktil, anzuzeigen.

Gemäß einer Ausführungsform umfasst die Bestimmungseinrichtung eine Mehrzahl von an einem Kopf eines Benutzers anordnenbaren Positionssensoren zur Positionsbestimmung und Ausgabe von Positionssignalen, und eine Mehrzahl von diskreten Leuchtelementen, insbesondere LEDs, zum Anzeigen des Hinweises auf eine Drehrichtung des Kopfes in der Transversalebene.

Gemäß einer Ausführungsform umfasst die Bestimmungseinrichtung zumindest eine Positionsbestimmungseinrichtung, insbesondere einen Positionssender, zur Erfassung der Ist-Position. Ein derartiger Positionssender kann beispielsweise an oder in einem der vorgenannten Skier untergebracht werden. Generell ermöglicht diese Ausführungsform, dass das erfindungsgemäße System durch einen Benutzer getragen werden kann.

Gemäß einer Ausführungsform umfasst das System ferner einen Objektgeber, insbesondere einen Ballgeber oder einen Puckgeber, zum Ausgeben des Objektes.

Gemäß einer Ausführungsform umfasst das System eine Mehrzahl der Erfassungseinrichtungen zur Erfassung der Ist-Position.

Gemäß einem weiteren Aspekt betrifft die Erfindung ein Verfahren zur Unterstützung einer Bewegungsübung mit einem Objekt, mit Erfassen einer Ist-Position des Objektes, Bestimmen einer Soll-Position des Objektes, und Anzeigen eines Hinweises auf die Soll-Position, wenn sich die Ist-Position von der Soll-Position unterscheidet.

Weitere Merkmale des Verfahrens ergeben sich unmittelbar aus der Funktionalität des Systems oder eines Merkmals des Systems.

Gemäß einer Ausführungsform kann das Verfahren durch das System ausgeführt werden.

Gemäß einem weiteren Aspekt betrifft die Erfindung ein Computerprogramm mit einem Programmcode zum Ausführen des erfindungsgemäßen Verfahrens, wenn der Programmcode auf einem Computer ausgeführt wird.

Weitere Ausführungsbeispiele der Erfindung werden Bezug nehmend auf die beiliegenden Zeichnungen näher erläutert. Es zeigen:
- Fig. 1: ein Blockdiagramm eines Systems zur Unterstützung einer Bewegungsübung mit einem Objekt gemäß einer Ausführungsform;
- Fig. 2: ein System zur Unterstützung einer Bewegungsübung gemäß einer Ausführungsform;
- Fig. 3: ein System zur Unterstützung einer Bewegungsübung gemäß einer weiteren Ausführungsform;
- Fig. 4: ein System zur Unterstützung einer Bewegungsübung gemäß einer weiteren Ausführungsform;
- Fig. 5: ein System zur Unterstützung einer Bewegungsübung gemäß einer weiteren Ausführungsform;
- Fig. 6: ein System zur Unterstützung einer Bewegungsübung gemäß einer weiteren Ausführungsform;
- Fig. 7: ein System zur Unterstützung einer Bewegungsübung gemäß einer weiteren Ausführungsform;
- Fig. 8: ein System zur Unterstützung einer Bewegungsübung gemäß einer weiteren Ausführungsform;
- Fig. 9: ein System zur Unterstützung einer Bewegungsübung gemäß einer weiteren Ausführungsform;
- Fig. 10: ein System zur Unterstützung einer Bewegungsübung gemäß einer weiteren Ausführungsform;
- Fig. 11: ein System zur Unterstützung einer Bewegungsübung gemäß einer weiteren Ausführungsform;
- Fig. 12: ein System zur Unterstützung einer Bewegungsübung gemäß einer weiteren Ausführungsform; und
- Fig. 13: ein System zur Unterstützung einer Bewegungsübung gemäß einer weiteren Ausführungsform.

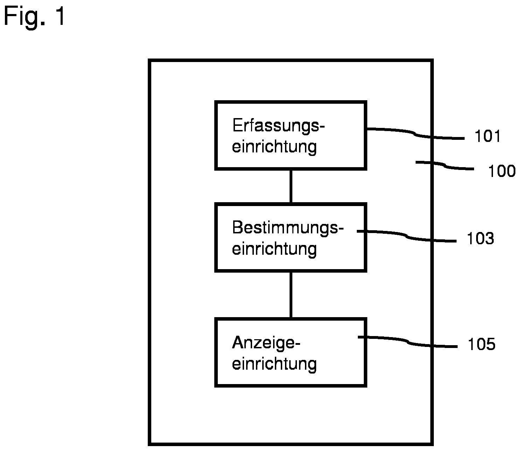

Fig. 1 zeigt ein Blockdiagramm eines Systems 100 zur Unterstützung einer Bewegungsübung mit einem Objekt. Das System 100 umfasst eine Erfassungseinrichtung 101 zum Erfassen einer Ist-Position des Objektes, eine Bestimmungseinrichtung 103 zum Bestimmen einer Soll-Position des Objektes und eine Anzeigeeinrichtung 105 zum Anzeigen eines Hinweises auf die Soll-Position, wenn, beispielsweise nur wenn, sich die Ist-Position von der Soll-Position unterscheidet.

In der nachstehenden Tabelle 1 sind einige Ausführungsbeispiele von Objekten für verschiedene Sportarten angeführt.

**Tabelle 1**

| | |
|---|---|
| - Sportart bzw. Disziplin | - Objekt: |
| - Badminton | - Badmintonschläger |
| - Basketball | - Basketball |
| - Bogenschießen | - Bogen, Pfeil |
| - Boxen | - Boxhandschuhe, Boxhose, Boxsack, Boxbirne |
| - Fechten | - Säbel, Helm, Anzug |
| - American Football | - Football |
| - Fußball | - Ball |
| - Gewichtheben | - Stange, Gewichte |
| - Golf | - Schläger, Schuhe, Anzug |
| - Handball | - Ball |
| - Hockey | - Hockeyschläger, Ball |
| - Judo | - Gürtel, Anzug |
| - Kanusport - Kanurennsport | - Kanu, Stangen im Wasser, Tore |
| - Kanusport - Kanuslalom | - Kanu, Stangen im Wasser, Tore |
| - Leichtathletik | - Diskus, Speer, Hammer |
| - Moderner Fünfkampf | - Pistole, Degen, Badehose, Bademütze, Pferd, Sattel, Reithut, Hufeisen, Hindernis, Laufschuh |
| - Radsport - Bahnradrennen | - Rad, Rahmen, Lenker, Räder, Sattel |
| - Radsport - BMX | - Rad, Rahmen, Lenker, Räder, Sattel |
| - Radsport - Mountainbike (Cross-Country) | - Rad, Rahmen, Lenker, Räder, Sattel |
| - Radsport - Straßenrennen | - Rad, Rahmen, Lenker, Räder, Sattel |
| - Reitsport - Dressur | - Sattel, Hindernisse, Reithut, Gürtel, Hufeisen |
| - Reitsport - Military | - Sattel, Hindernisse, Reithut, Gürtel, Hufeisen |
| - Reitsport - Springen | - Sattel, Hindernisse, Reithut, Gürtel, Hufeisen |
| - Ringen - Freistil | - Anzug, Gürtel |
| - Ringen - griechisch-römisch | - Anzug, Gürtel |
| - Rudern | - Ruder, Boot |
| - Rugby (7er) | - Ball, Helm, Anzug |
| - Schießen | - Bogen, Pfeil |
| - Schwimmsport - Kunst- und Turmspringen | - Badehose |
| - Schwimmsport - Schwimmen | - Badehose |
| - Schwimmsport - Synchronschwimmen | - Badehose |
| - Schwimmsport - Wasserball | - Ball, Tore |
| - Segeln | - Mast, Boot, Ruder |
| - Taekwondo | - Anzug |
| - Tennis | - Tennisschläger |
| - Tischtennis | - Ball, Tischtennisschläger |
| - Triathlon | - Rad, Schuhe, Badehose |
| - Turnsport - Kunstturnen | - Anzug |
| - Turnsport - Rhythmische Sportgymnastik | - Ball, Stab |
| - Turnsport - Trampolin | - Tuch, |
| - Volleyball - Beachvolleyball | - Volleyball |
| - Volleyball - Volleyball | - Volleyball |
| - Biathlon | - Gewehr, Ski, Skischuhe, Skianzug |
| - Bobsport - Bobfahren | - Bob, Helm |
| - Bobsport - Skeleton | - Bob, Helm |
| - Curling | - Curl |
| - Eishockey | - Puck, Anzug, Helm, Tor |
| - Eislauf - Eiskunstlaufen | - Schlittschuh, Anzug |
| - Eislauf - Shorttrack | - Schlittschuh, Anzug |
| - Eislauf - Eisschnelllauf | - Schlittschuh, Anzug |
| - Rennrodeln | - Schlitten, Helm |
| - Skisport - Ski Alpin | - Ski, Bindung, Schuh |
| - Skisport - Freestyle-Skiing | - Ski, Bindung, Schuh |
| - Skisport - Ski Nordisch - Nordische Kombination | - Ski, Bindung, Schuh |
| - Skisport - Ski Nordisch - Skilanglauf | - Ski, Bindung, Schuh |
| - Skisport - Ski Nordisch - Skispringen | - Ski, Bindung, Schuh |
| - Skisport - Snowboard | - Snowboard |

In der nachstehenden Tabelle 2 sind einige Ausführungsbeispiele von personenbezogenen Objekten angeführt.

**Tabelle 2**

| |
|---|
| Kopf, Stirn, Hinterkopf |
| Nasion Inion, präaurikuläre Region |
| HWS, BWS, LWS |
| Brustbein |
| Schulterhöhe (Acromion) |
| Ellenbogengelenk |
| Handgelenk |
| Spina Iliaca anterior superior |
| Trochanter major (großer Rollhügel) |
| Kniegelenk |
| Oberes Sprunggelenk |
| Fußspitze |
| Ferse |
| Bauchnabel |

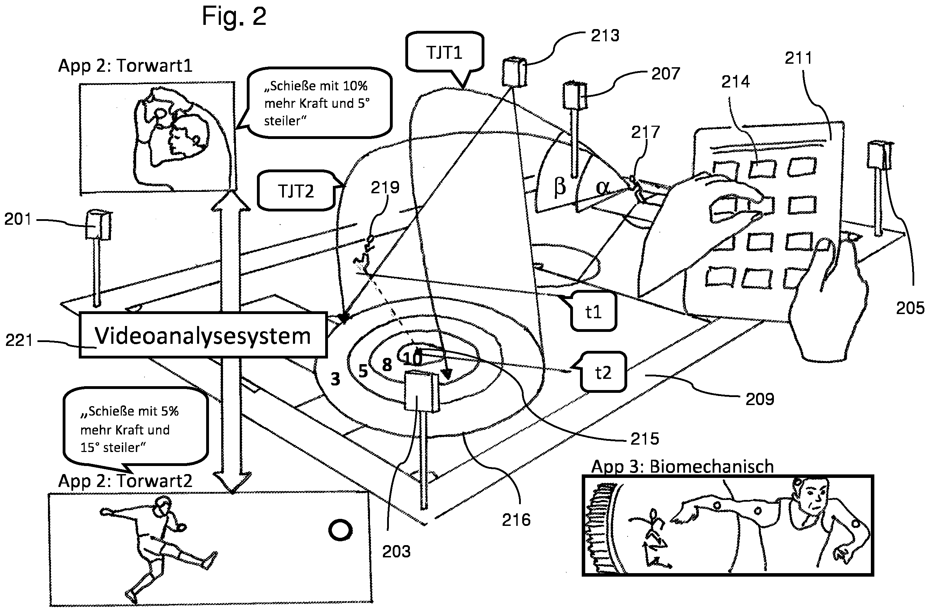

Fig. 2 zeigt ein System zur Unterstützung einer Bewegungsübung mit einem Objekt, das beispielsweise ein Fußball ist. Das System umfasst ein Positionslokalisierungssystem mit einer Mehrzahl von Erfassungseinrichtungen 201, 203, 205 und 207 (PLS1, PLS2, PLS3, PLS4), welche beabstandet voneinander, beispielsweise in Ecken eines Fußballfeldes 2109, angeordnet sind. Das System kann jedoch nur eine, zwei oder drei oder mehr als 4 Erfassungseinrichtungen umfassen.

Das System umfasst ferner eine Bestimmungseinrichtung 211 zum Bestimmen einer Ist-Position des Objektes, welche der gegenwärtigen Ist-Position des Objektes entspricht. Die Bestimmungseinrichtung kann beispielsweise auf einem Smartphone mittels eines Applikationsprogramms realisiert werden, mit mehreren Applikationsfeldern 214, welche jeweils eine Auswahl einer Soll-Position ermöglichen. Die Bestimmungseinrichtung 211 kann jedoch auch ein separater Rechner oder ein Rechnercluster sein.

Das System umfasst ferner eine Anzeigeeinrichtung 213 zum Anzeigen eines Hinweises auf die Soll-Position, sofern sich die Ist-Position von der Soll-Position unterscheidet. Die Soll-Position 215 kann beispielsweise in einem Bereich 216 des Spielfeldes 209 liegen. Die Anzeigevorrichtung 213 kann beispielsweise ausgebildet sein, konzentrische Kreise um die Soll-Position 215 auf das Spielfeld zu projizieren, um beispielsweise einem Torwart 217 die Soll-Position anzuzeigen. Die Ist-Position des Objektes kann beispielsweise der Ist-Position des Torwarts 217 entsprechen. Befindet sich beispielsweise auf dem Spielfeld 209 ein weiterer Spieler 219, so kann die Soll-Position 215 in Abhängigkeit von einer Ist-Position des weiteren Spielers 219 bestimmt und/oder angezeigt werden. Hierzu kann der weitere Spieler 219 mit einem Sender ausgestattet sein, welcher eine Position des weiteren Spielers 219 an die Erfassungseinrichtungen 201 bis 205, welche eine gemeinsame Erfassungseinrichtung bilden, aussendet.

Das System kann ferner ein Videoanalysesystem 221 umfassen, dass als Applikation das Verhalten eines ersten Torwarts und eines zweiten Torwarts anzeigen kann.

Um mit einem weiten Abschlag aus der Hand zielgenau einen Ball in den Lauf eines Angreifers spielen zu können sind verschiedene Teilfertigkeiten eines Torhüters (TW1) vorteilhaft. Zunächst ist dies die Antizipationsfähigkeit des Torhüters im Hinblick auf die zu erwartende Position eines Mitspielers zum Zeitpunkt der Ballannahme durch den Mitspieler P1 in Position 10. Weiterhin sollte der Torhüter in Bezug auf sein technisches, Kraft- und Koordinationsniveau dazu in der Lage sein, den Ball adäquat mit folgenden biomechanischen Einflussgrößen anzusteuern:
- Linearen Impuls
- Drehimpuls (links, rechts)
- Abschlagwinkel (horizontal, vertikal)
Die hier genannten biomechanischen Einflussgrößen sind ebenso wie die Torhüterbewegung in Echtzeit in an sich bekannter Weise erfassbar.

Beginnt nun der anzuspielende Spieler P1 zum Zeitpunkt t1 mit der mittleren Geschwindigkeit v in eine bestimmte Richtung zu laufen, so kann das System basierend auf Modellannahmen, welche deterministische biomechanische Gesetzmäßigkeiten umfassen und in der Datenbank hinterlegt sein können, vorausberechnen wo der Ball zum Zeitpunkt t2 landen würde, wenn er beispielsweise mit vorbekannten biomechanischen Eingangsvoraussetzungen abgeschlagen wird um zum Zeitpunkt t2 in der Nähe des Fußes des Spielers zu landen. Dieses anzusteuernde Ziel in der Gestalt einer Zielscheibe kann permanent oder dynamisch veränderbar von der Anzeigeeinrichtung 213 auf die Spiel- bzw. Trainingsfläche projiziert werden. Der Torhüter könnte somit schon bevor er den Ball final abschlägt per Echtzeitfeedback die Zielzone visualisiert bekommen. Ebenso stehen in geringer Zeitverzögerung (0,1s-0,5s) jene Parameter zur Verfügung unter deren Einfluss die Balltrajektorie zu Stande gekommen ist, mit Abschlagwinkel alpha bzw. beta, Impuls des Balles p, Balltrajektorie, etc. In Echtzeit kann mit dem System auch die Zielpräzision des Abschlages angegeben werden, beispielsweise innerhalb der Zielscheibe. Im Lern- bzw. Echtzeitrückkopplungsmodus kann der Torhüter nun entscheiden auf welcher Grundlage die Rückkopplung erfolgen soll. Hierzu wählt der Torhüter oder Trainer zwischen verschiedenen Applikation (Apps) auf einem Tablet-PC, PC, Notebook, Smartphone oder sonstigem Anzeigegerät aus. Er kann entscheiden, ob die Soll/ Istwert Abweichung auf der Basis des statistischen Datenmaterials das die Datenbank über einen Torhüter, über den die Datenbank Daten enthält, erfolgt. Alternativ kann er sich auch in Referenz zu unterschiedlichen biomechanischen Modellen in eine Echtzeit-Trainings- und Lerneinheit begeben. Er kann dann auswählen, ob die Ausführung des Abschlages weitenoptimiert, präzisionsoptimiert oder zeitoptimiert erfolgen soll.

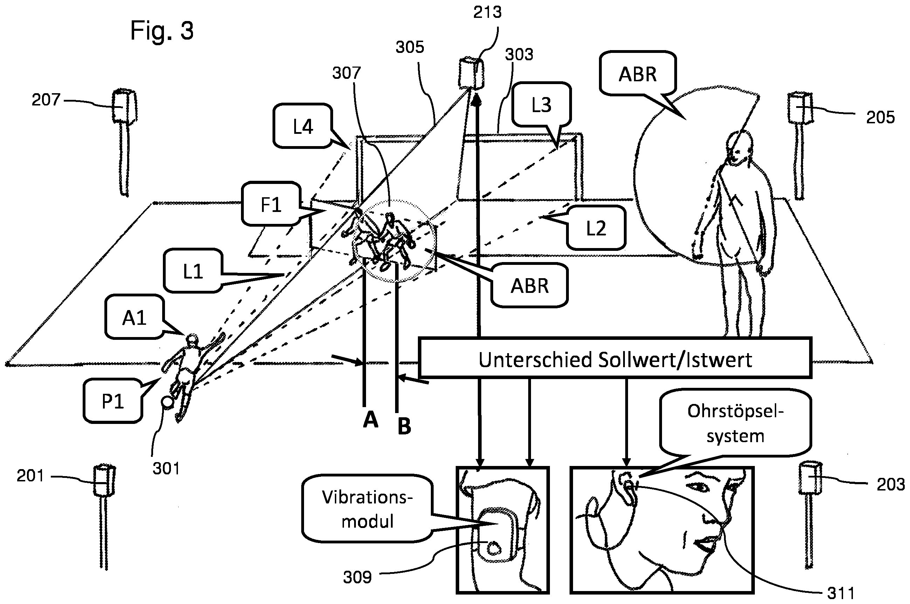

Fig. 3 zeigt ein System zur Unterstützung einer Bewegungsausübung mit einem Objekt, beispielsweise mit einem Fußball, gemäß einer Ausführungsform. Das System umfasst zumindest eine der in Fig. 2 dargestellten Erfassungseinrichtungen 201 bis 207. Diese erfassen beispielsweise eine Ist-Position eines Objektes 301, welches ein Ball sein kann. Die Bestimmungseinrichtung, welche in Fig. 3 nicht dargestellt ist, ermittelt beispielsweise anhand der Ist-Position des Balls 301 und der Mitte eines Tors 303 eine Verbindungslinie 305 zwischen Tormitte und der Ist-Position des Balls 301.

Die Anzeigeeinrichtung 213 kann ausgebildet sein, die Verbindungslinie 305 auf das Spielfeld zu projizieren und einem Torwart 307, welcher gemäß einer Ausführungsform ein Objekt im Sinne der vorliegenden Beschreibung sein kann, als Soll-Position anzuzeigen.

Gemäß einer Ausführungsform kann die Anzeigeeinrichtung 213 alternativ oder zusätzlich zumindest ein Vibrationsmodul 309 umfassen, welches dem Torwart die Richtung zur Verbindungslinie 305 anzeigt. Hierzu kann der Torwart beidseitig, beispielsweise an den Oberarmen, Vibrationsmodule tragen, welche in Abhängigkeit von der Ist-Position des Torwarts 307 einen Vibrationshinweis in Richtung der Verbindungslinie 305 anzeigen.

Gemäß einer Ausführungsform kann die Anzeigevorrichtung 213 alternativ oder zusätzlich zumindest einen Lautsprecher 311 umfassen, welcher beispielsweise in der Gestalt eines Ohrsteckers, beispielsweise am Ohr des Torwarts, befestigt werden kann. Der Lautsprecher 311 ist ausgebildet, akustische Signale auszugeben, welche auf die Lage der Soll-Position, d.h. welche auf die Lage der Verbindungslinie 305 hinweisen. Gemäß einer Ausführungsform kann so auf die Projektion der Verbindungslinie auf das Spielfeld verzichtet werden. Gemäß einer anderen Ausführungsform können zumindest zwei der vorgenannten Ausführungsformen, beispielsweise Projektion und Vibration oder Projektion und akustisches Signal, gemeinsam eingesetzt werden.

Schießt beispielweise der Angreifer A1 aus der Position P1 aufs Tor, so geht bei einer annähernd geradlinigen Ballflugbahn der Ball aus geometrischen Gründen nur dann ins Tor, wenn er sich innerhalb eines Raumes bewegt, der sich zwischen dem Ball und den gedachten Verbindungslinien (L1-L4) aufspannt. Die individualtaktische Aufgabe des Torhüters besteht nun darin durch seine Körperhaltung und Position mit seiner anthropometrisch bedingten Reichweite (ABR) eine möglichst große Teilfläche der Fläche (F1) abzudecken. Dies gelingt ihm dann am besten, wenn er auf der gedachten Linie zwischen Ballmitte und Tormitte agiert. Diese Linie kann mit der Anzeigeeinrichtung 213, welche ein optisch-akustisch-taktiles Feedbacksystem bilden kann, während des Schusstrainings bzw. Spiels auf das Spiel- bzw.

Trainingsfeld projiziert werden. Da die Ortsposition des Balles 301 in Echtzeit an das System übermittelt werden kann, wandert diese Linie permanent mit der Ballposition mit. Der Torhüter kann somit via visuellem Echtzeitfeedback sein individualtaktisches Stellungsspiel permanent optimieren. Parallel zum visuellen Echtzeitfeedback erfolgt ein akustisches Feedback über ein Minikopfhörersystem und/oder ein kleines Lautsprechersystem, das am Körper des Torhüters befestigt ist und/oder über eine Beschallungsanlage. Dieses Audiofeedback funktioniert derart, dass beispielsweise bei der Einnahme der korrekten Position der Kammerton a eingespielt wird und bei größer werdender Soll-/Istwert Differenz (SID) die Frequenz proportional zur Entfernung von der Idealposition verändert wird. Ebenso könnte das System von der akustischen Rückmeldung funktionieren wie das Echolot oder wie eine akustische Rückmeldung einer Einparkhilfe, etc. Die dritte Komponente des Echtzeitfeedback erfolgt in Form einer Soll/Istwert Differenz gesteuerten Veränderung der Vibrationsfrequenz und/oder Amplitude eines taktilakustischen Feedbacksystems, das am Körper des Torhüters getragen werden und ein Element der Anzeigeeinrichtung 213 sein kann.

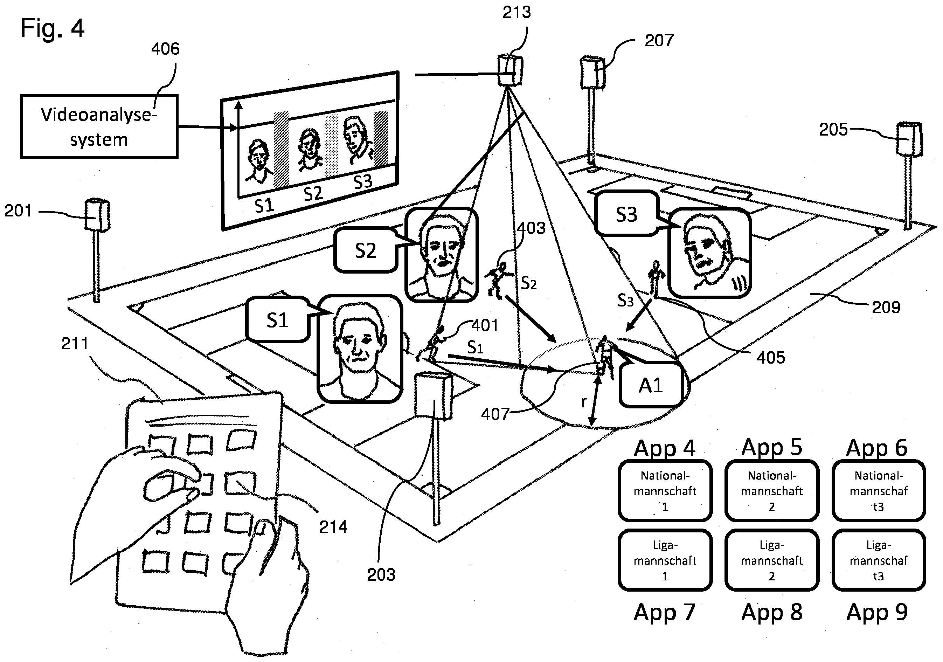

Fig. 4 zeigt ein System zur Unterstützung einer Bewegungsübung gemäß einer weiteren Ausführungsform. Die auf dem Spielfeld 209 befindlichen Spieler 401 (S1), 403 (S2), 405 (S3) und 407 (A1) führen eine Übung mit einem Ball 409 durch, welcher sich beispielsweise bei dem Spieler 407 befindet, der ein Angreifer ist. Die Spieler 401, 403 und 405 können beispielsweise als Objekte im Sinne der vorliegenden Beschreibung verstanden werden. Die Anzeigeeinrichtung 213 kann beispielsweise Signale erzeugen, welche den Spielern 401 bis 405 oder nur einem der Spieler 401, 403, 405 ein Signal übermitteln, welches einen Hinweis auf dessen Soll-Position enthält. Der Hinweis auf die Soll-Position kann dem jeweiligen Spieler 401, 403, 405 taktil, akustisch oder durch eine Vibration angezeigt werden. Hierzu kann die Erfassungseinrichtung 213 beispielsweise ein Vibrationsmodul oder einen Lautsprecher aufweisen, welche an den jeweiligen Spielern 401 bis 405 angebracht sein können. Das System kann ferner ein Videoanalysesystem 406 umfassen.

In dem in Fig. 4 dargestellten Ausführungsbeispiel ist die Gruppentaktik gegen den Ball mit sogenanntem Pressing verdeutlicht.

Im modernen Fußball beginnt das verteidigen bereits tief in der gegnerischen Platzhälfte. Kommt z.B. ein Angreifer der gegnerischen Mannschaft (A1) in Ballbesitz, so besteht die Aufgabe der ihn umgebenden Gegenspieler (S1-S3) darin, den Spieler A1 so aggressiv wie möglich zu attackieren, um wieder selbst in Ballbesitz zu kommen. Dies nennt man Pressing. Die Aggressivität, mit der ein Spieler presst ist durch die Höhe der Beschleunigung messbar und beurteilbar. Liegen nun in der Datenbank Referenzwerte bezüglich des Beschleunigungsverhaltens in Pressingsituationen der National- und Klubmannschaften und der weltbesten Fußballer vor, so können diese genutzt werden, um als Sollwerte in einem Echtzeitfeedback - Training, -Coaching und -Learning eingesetzt werden zu können. Der Ablauf in einer Echtzeitfeedback-Trainingssituation wäre gemäß einer Ausführungsform der Folgende:
1) Ein Trainer wählt auf Tablet-PC, Smartphone, PC Notebook oder sonstiger Einstell- und Displayeinheit der Bestimmungseinrichtung 211 aus, mit welchen Referenzwerten aus der Datenbank der Abgleich erfolgen soll, um zu den Soll-/Istwertdifferenzen zu gelangen. Die Apps 4,5,6 referenzieren auf die Normwerte von Nationalteams und die Apps 7,8,9 auf die Referenzwerte von Klubmannschaften.
2) Der Trainer hält beispielsweise einen Drucktaster in der Hand, mit dem er den Zeitpunkt zu dem Pressing von der Trainingsgruppe gespielt werden soll steuern kann. Drückt der Trainer diesen Taster, so wird durch die Anzeigeeinrichtung 213 ein Lichtkegel mit dem Radius r um den ballführenden Spieler projiziert, ebenso erfolgt ein Signalton über die Beschallungsanlage und/oder Minikopfhörer und/oder am Körper befindliche Lautsprechersysteme. Zusätzlich erfolgt je nach Setup auch eine taktile Rückmeldung über ein in Frequenz und Amplitude steuerbares Vibrationssystem.
3) Läuft nun einer der Spieler mit zu stark von den Referenzwerten abweichenden Beschleunigungswerten auf den ballführenden Gegenspieler zu, so kann dies wiederum durch die beschriebenen Echtzeitfeedbackmethoden an den Spieler zurückgemeldet werden.
4) Ist die Aktion vorüber, so kann das die Anzeigeeinrichtung 213 die Sollwerte, Istwerte und die Soll-Istwert Differenz auf eine Fläche projizieren und/oder das Ergebnis auf einem Tablet-PC und/oder einem Smartphone oder einer sonstigen Steuer- und Visualisierungseinheit anzeigen.

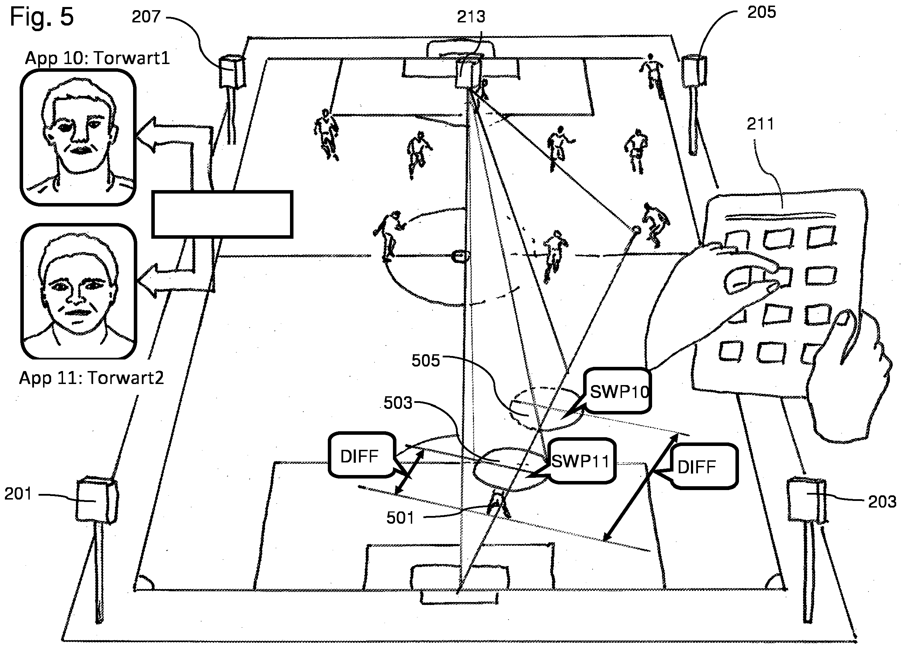

Fig. 5 zeigt ein System gemäß einer weiteren Ausführungsform, bei dem beispielsweise einem Torhüter 501 eine Mehrzahl von Hinweisen auf Soll-Positionen 503, 505 angezeigt werden können.

Im modernen Fußballspiel soll der Torhüter hinter der hinteren Verteidigungskette als mitspielender Torhüter agieren. Als Grundregel gilt hierbei, dass der Torhüter sich auf der gedachten Verbindungslinie Tormitte-Ballmitte mitbewegen sollte. In einem Echtzeitfeedback-Trainings- bzw. Spielszenario könnte nun diese Verbindungslinie durch die Anzeigeeinrichtung 213 während des Trainings- bzw. Spielgeschehens permanent auf die Spiel- bzw.

Trainingsfläche projiziert werden. Durch diese Echtzeitfeedback-Projektion könnten Torhüter sehr schnell lernen, sich auf der gedachten Linie Tor-Ball zu bewegen. Neben der visuellen Rückmeldung könnte auch eine akustische und taktile Rückmeldung erfolgen, falls die Soll-/Istwert Differenzen bestimmte Grenzwerte überschreiten. Der Trainer, bzw. Torhüter hat beispielweise die Möglichkeit zu entscheiden, in Bezug auf welche in der Datenbank hinterlegten statistischen Normwerte die Soll/Istwert Differenz berechnet werden soll. Hierzu werden in der Datenbank aus der Analyse von Videosequenzen des typischen Verhaltens eines bestimmten Torhüters Werte hinterlegt, die in an sich bekannter Weise gewonnen werden können. Die Sollwertposition des Torhüters wird beispielsweise so bestimmt, dass der durchschnittliche Abstand des Torhüters zum geometrischen Schwerpunkt der Mannschaft empirisch-statistsich zum Beispiel aus 100 typischen Videoszenen der Vergangenheit ermittelt und in der Datenbank hinterlegt wird. Dieser Sollwert wird nun mit dem permanent zu erfassenden Schwerpunkt des trainierenden oder spielenden Teams in eine mathematische Beziehung gesetzt und das Ergebnis in Echtzeit visuell während des Trainings oder Spiels dargestellt. So würde die Sollwertposition des Torhüters, die über die App 10 eingeschaltet werden kann in Form einer Kreisfläche (SWP 10) in Echtzeit dynamisch auf das Spiel- oder Trainingsfeld projiziert. Analog könnte auch das typische Verhalten eines anderen Torhüters in Echtzeit auf dem Platz dargestellt werden (SWP 11).

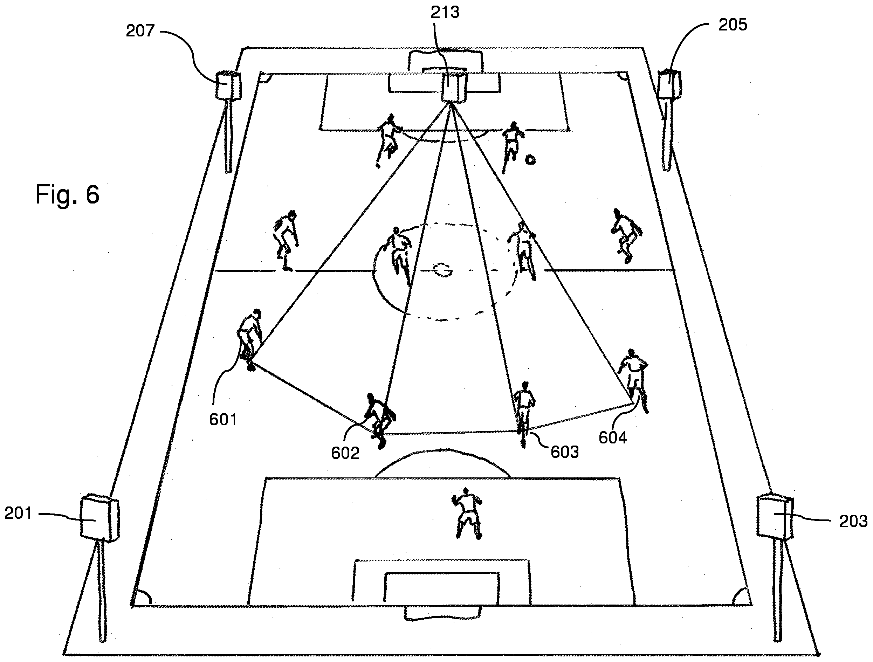

Fig. 6 zeigt ein System gemäß einer weiteren Ausführungsform, bei dem die Spieler 601-604 Objekte im Sinne der vorliegenden Beschreibung sind. Dabei wird zumindest einem oder mehreren der Spieler 601 ein Hinweis auf dessen individuelle Soll-Position in Abhängigkeit von Ist-Positionen der übrigen Spieler angezeigt. Hierbei kann beispielsweise ein Schwerpunkt, beispielsweise ein geometrischer Schwerpunkt, aus den Ist-Positionen der übrigen Spieler bestimmt werden, um den jeweiligen Spieler 601 dessen individuelle Soll-Position anzuzeigen.

Gemäß einer Ausführungsform kann auf diese Weise der Abstand zwischen den Spielern überwacht werden. Der Abstand von Spieler 601 zu Spieler 602 ist beispielsweise zu groß, so dass ein Hinweis ausgegeben werden kann, diesen Abstand zu verkleinern, so dass er dem Soll-Abstand als Soll-Position entspricht. Der Soll-Abstand ist beispielsweise vorgegeben oder er ergibt sich aus Modellansätzen, die deterministisch und/oder empirisch-statistisch sein können, welche die Sollposition der Spieler in Echtzeit dynamisch verfügbar vorhalten.

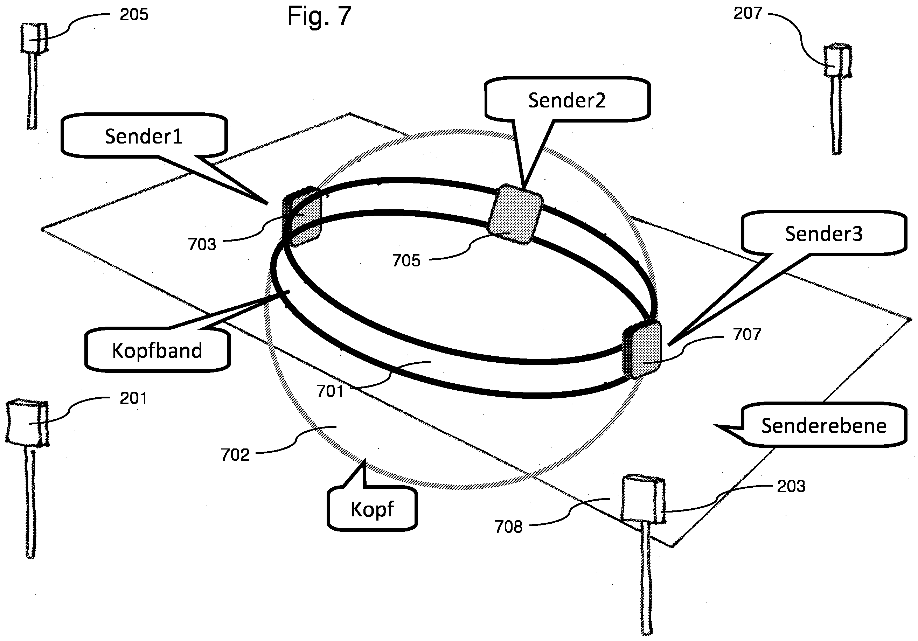

Fig. 7 zeigt ein System gemäß einer Ausführungsform, bei dem eine Anzeigeeinrichtung 701 beispielsweise in einem an einem Kopf eines Spielers anordnenbaren oder anbringbaren Kopfband angeordnet ist. Der Kopf des Spielers kann beispielsweise als Objekt im Sinne der vorliegenden Beschreibung verstanden werden. Die Anzeigeeinrichtung 701 kann beispielsweise drei Sender 703, 705, 707 umfassen, welche Sendesignale an die Erfassungseinrichtungen 201, 203, 205 und 207 aussenden und in einer Senderebene 708 angeordnet sind. Die Erfassungseinrichtungen 201 bis 207 erfassen auf der Basis dieser Signale die Ist-Position des Kopfes 702, beispielsweise eine Drehposition des Kopfes. Eine in Fig. 7 nicht dargestellte Bestimmungseinrichtung kann auf der Basis der gegenwärtigen Ist-Position des Kopfes 702 beispielsweise einen Hinweis auf eine Soll-Position des Kopfes 702 erzeugen, da dieser beispielsweise noch stärker nach rechts gedreht werden soll. Dies kann beispielsweise optisch, akustisch oder taktil angezeigt werden. Gemäß einer Ausführungsform können nur eine, zwei oder drei oder mehrere der Erfassungseinrichtungen 201 bis 207 vorgesehen sein.

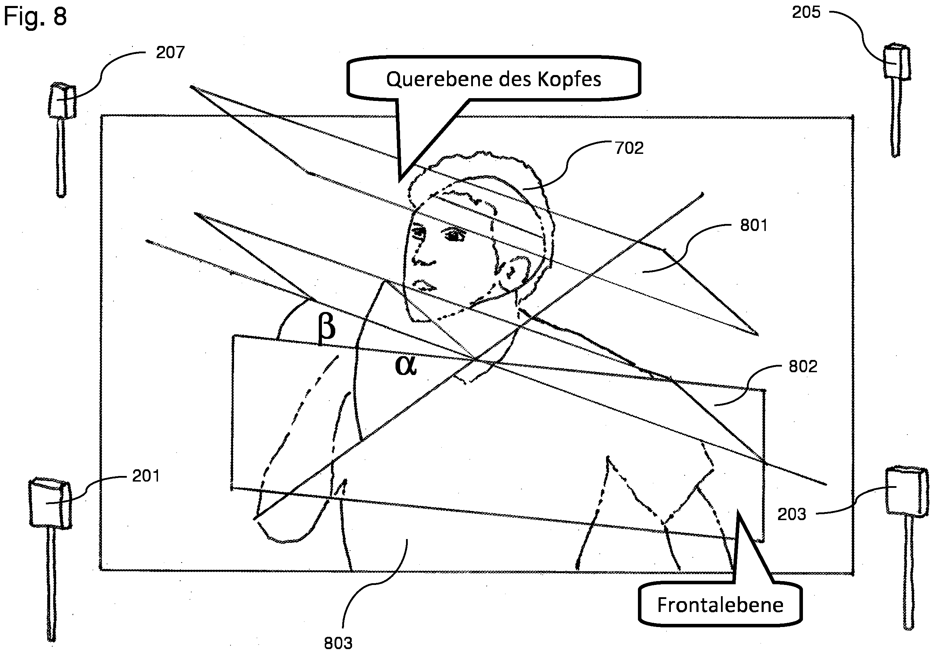

Fig. 8 verdeutlicht die Wirkungsweise des in Fig. 7 dargestellten Systems mit einer Transversalebene 801 des Kopfes 702, welcher der Senderebene 801 entsprechen kann sowie mit einer Frontalebene 802 des Spielers 803. Die Anzeigeeinrichtung kann beispielsweise eine Drehung des Kopfes 702 in der Transversalebene 801 bzw. eine Stellung des Kopfes 702 bezüglich der Frontalebene 802 anzeigen.

Gemäß einer Ausführungsform berechnet und erfasst das Positionslokalisierungssystem mit den Erfassungseinrichtungen 201, 203, 205, 207, etc. die Ist-Positionen drei unterschiedlicher Stellen des Kopfes eines Menschen in Echtzeit. Hieraus berechnet das System die Transversalebene des Kopfes. Ebenso erfasst und das System drei unterschiedliche Positionen des Rumpfes des Menschen und berechnet daraus die frontale Rumpfebene des Menschen in Echtzeit. Das System berechnet aus der Stellung der beiden Ebenen die Winkel alpha und beta.

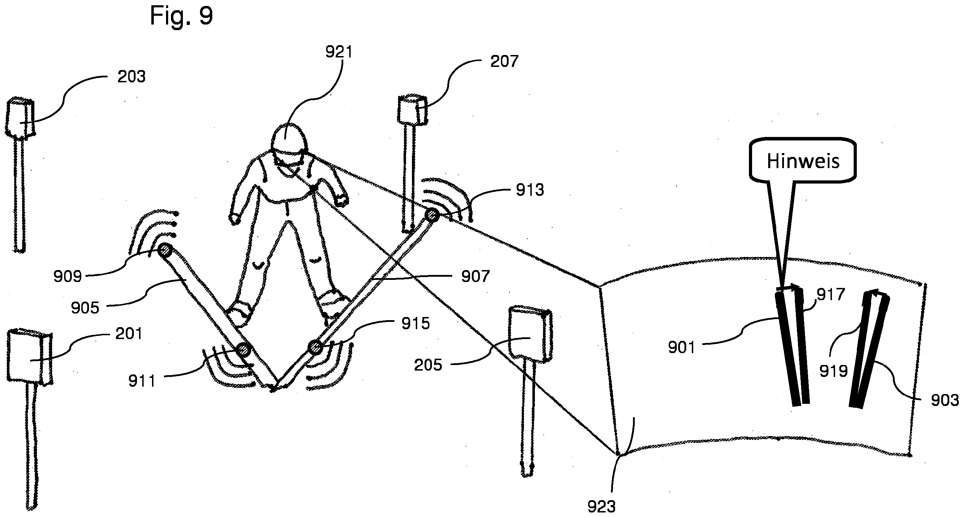

Fig. 9 zeigt ein System zur Unterstützung einer Bewegungsübung beim Skispringen, bei dem die Ist-Positionen 901, 903 der Skier mittels der Erfassungseinrichtungen 201, 205, 207 und 209 erfasst werden. Hierzu kann jeder Ski 905, 907 mit einem oder mehreren Positionssensoren 909, 911, 913, 915 versehen werden. Die Positionssensoren können beispielsweise auf den Skiern 905, 907 paarweise angeordnet sein, wobei einer der Positionssensoren 909, 913 jeweils im vorderen Bereich des Skis, beispielsweise an einer Skispitze, und wobei der andere Positionssensor 911, 915 jeweils beispielsweise im Fersenbereich des Skis oder im hinteren Bereich des Skis angeordnet ist, vorzugsweise am Skiende. Auf der Basis der Ist-Positionen 901, 903 können Soll-Positionen 917, 919 berechnet und mittels einer Anzeigeeinrichtung, welche beispielsweise in einem Helm 921 integriert sein kann, auf ein Helmvisier 923 projiziert werden. So kann der Skispringer während des Skiflugs die Stellung der Skier 905, 907 bezüglich einander oder bezüglich der Soll-Position korrigieren.

Gemäß einer Ausführungsform erfasst und berechnet das Positionslokalisierungssystem mit den Erfassungseinrichtungen 201, 203, 205, 207 die Stellung der Skier während des Skisprungs in Echtzeit. Die Anlage sendet in Echtzeit die Ist-Position in Form einer roten Linie auf ein Spezialvisier, das in den Sprunghelm integriert ist. Gleichzeitig projiziert das System die Sollposition ebenfalls auf das Visier. Die Soll-Position ist jene Position der Skier im Raum die auf Grund permanent erfasster Einflussgrößen gemäß einem biomechanischen Modell die höchste Sprungweite ergeben würde.

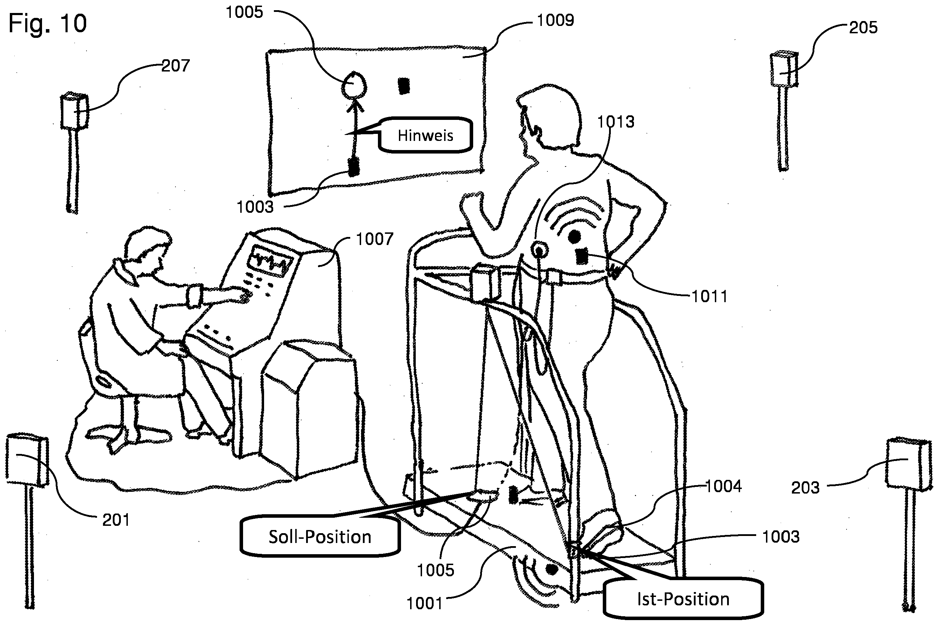

Fig. 10 zeigt ein System zur Unterstützung einer Bewegungsübung im Rehabilitationsbereich, bei dem beispielsweise einem Rehabilitationspatienten, welcher sich auf einem Laufband 1001 befindet, ausgehend von einer Ist-Position 1003 eines Fußes einem Hinweis auf eine Soll-Position des Fußes 1004, das als Objekt verstanden werden kann, gegeben wird. Hierzu kann eine beispielsweise in einer Konsole 1007 des Systems untergebrachte Bestimmungseinrichtung die Soll-Position 1005 bestimmen. Eine Anzeigeeinrichtung kann beispielsweise auf einem Display 1009 einen optischen Hinweis auf die Soll-Position 1005 des Fußes 1004 geben. Die Soll-Position kann alternativ oder zusätzlich auch auf das Laufband projiziert werden. Der Hinweis kann gemäß einer Ausführungsform taktil mittels eines Vibrators 1011 angezeigt werden, welcher beispielsweise am Körper des Rehabilitationspatienten befestigt werden kann. Hierdurch kann beispielsweise als Hinweis auf die Soll-Position ein Vibrationssignal erzeugt werden, dessen Vibrationsfrequenz beispielsweise von einer Differenz zwischen der Ist-Position 1003 und der Soll-Position 1005 abhängt.

Auf diese Weise kann der Rehabilitationspatient in seiner Bewegungsübung effizient unterstützt werden. Darüber hinaus kann der Patient mit einem Körperparametersensor 1013 versehen sein, welcher Körperparameter, wie etwa Herzfrequenz oder Pulsfrequenz, erfasst und an die Systemkonsole 1007 übermittelt. Die Bestimmungseinrichtung kann die Soll-Position 1005 in Abhängigkeit von dem zumindest einen Körperparameter bestimmen. Auf diese Weise kann die Bestimmung der Soll-Position 1005 an die körperlichen Gegebenheiten des Rehabilitationspatienten angepasst werden.

Gemäß einer Ausführungsform berechnet und erfasst das Positionslokalisierungssystem mit den Erfassungseinrichtungen 201, 203, 205, 207 die Stellung der Füße eines Patienten mit Bewegungsstörung, beispielsweise einen Zustand nach Apoplex, während des Gehens in Echtzeit. Die Anlage sendet in Echtzeit die Ist-Position der Fußspitze in Form roter Punkte auf den Boden des Laufbandes und virtuell auf einen Monitor der sich vor dem Patienten in Augenhöhe befindet. Gleichzeitig projiziert das System die Sollposition der Fußspitze in Form eines grünen Punktes ebenfalls auf das Laufband bzw. den Monitor. Die Sollposition ist jene Position welche der Patient bei submaximaler Anstrengung auf Grund der Verrechnung permanent erfasster Einflussgrößen gemäß einem biomechanischen Modell erreichen kann.

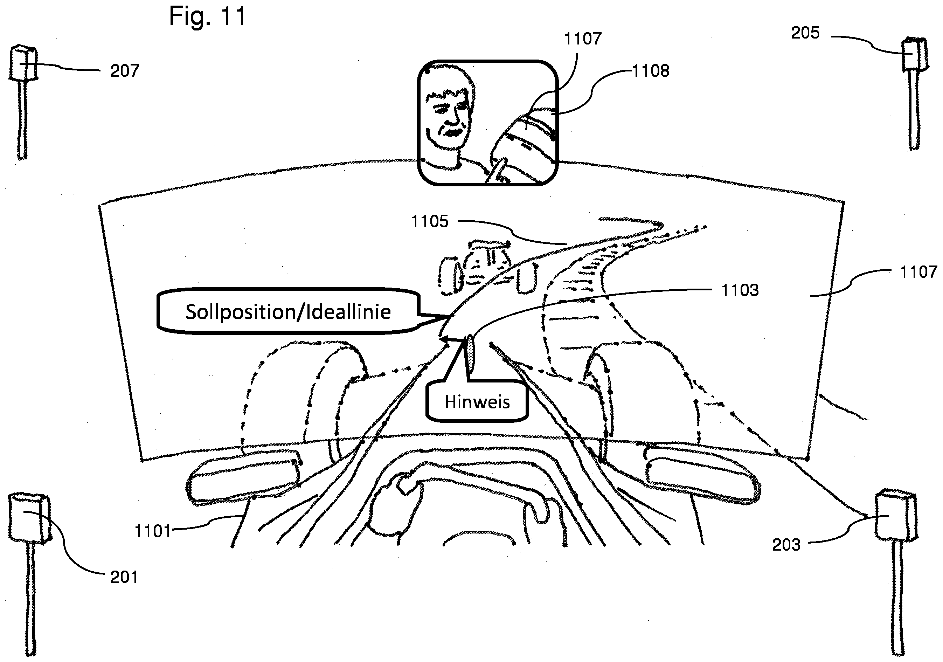

Fig. 11 zeigt ein System gemäß einer Ausführungsform mit einem Fahrzeug 1101, beispielsweise einem Rennfahrzeug, als Objekt. Das Fahrzeug 1101 kann beispielsweise einen Positionssender umfassen, dessen Sendesignale die Erfassungseinrichtungen 201 bis 207 empfangen. Eine in Fig. 11 nicht dargestellte Bestimmungseinrichtung kann auf dieser Basis in an sich bekannter Weise die aktuelle Ist-Position 1103 des Fahrzeugs 1001 ermitteln. Eine Anzeigeeinrichtung kann beispielsweise einen Projektor umfassen, welcher einen Hinweis auf die bestimmte Soll-Position 1105 beispielsweise in der Gestalt einer Fahr-Ideallinie auf ein Visier 1107 eines Fahrerhelms 1108 projiziert. Die Projektion kann jedoch auch auf eine Windschutzscheibe des Fahrzeugs geworfen werden.

Gemäß einer Ausführungsform berechnet und erfasst das Positionslokalisierungssystem mit den Erfassungseinrichtungen 201, 203, 205, 207 die Ist-Position eines Rennwagens während eines Rennens in Echtzeit. Die Anlage sendet in Echtzeit die Soll-Position des Wagens virtuell auf ein Spezialvisier, das in den Helm des Piloten integriert ist. Die Soll-Position ist beispielsweise jener Ort auf der Ideallinie, der zum Zeitpunkt der Erfassung der Ist-Position vom Fahrzeug eingenommen werden müsste, um die geringste Rundenzeit zu realisieren.

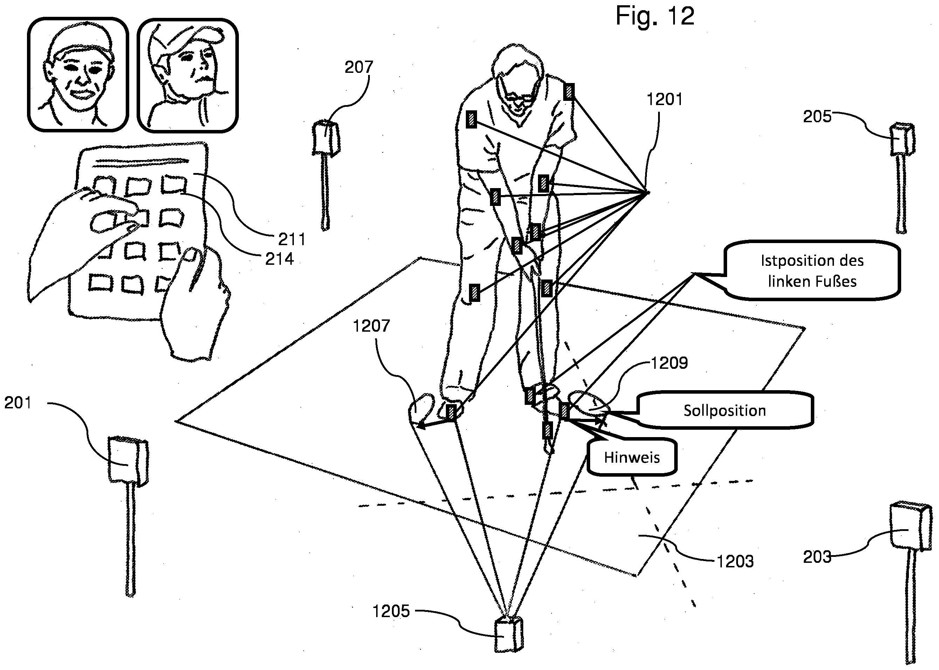

Fig. 12 zeigt ein System zur Unterstützung einer Bewegungsübung gemäß einer Ausführungsform, welche auf das Golfspiel gerichtet ist. Hierzu umfasst die Bestimmungseinrichtung eine Mehrzahl von Sendern 1201, welche an unterschiedlichen Körperteilen eines Golfspielers, beispielsweise an Knien, Handgelenken, Ellbogen oder Oberarmen, angeordnet sind. Die Erfassungseinrichtungen 201 bis 207 können auf der Basis der Sendesignale der Positionssensoren 201 die Stellung der einzelnen Körperglieder, wie etwa der Füße, als Ist-Positionen bestimmen. Der Spieler befindet sich beispielsweise in einem Spielbereich 1203, welcher beispielsweise als eine Standplatte ausgebildet sein kann. Eine Anzeigeeinrichtung 1205 kann beispielsweise Soll-Positionen 1207, 1209 der einzelnen Füße der Spieler auf die Standplatte 1203 von oben oder von unten projizieren. Gemäß einer Ausführungsform kann die Anzeigeeinrichtung 1205 in der Standplatte 1203 untergebrachte Leuchtelemente, wie etwa Licht emittierende Dioden, zum Anzeigen der Soll-Positionen 1207, 1209 anregen.

Gemäß einer Ausführungsform berechnet und erfasst das Positionslokalisierungssystem mit den Erfassungseinrichtungen 201, 203, 205, 207 die Ist-Positionen unterschiedlicher Stellen des Golfspielers in Echtzeit. Hierunter ist auch die Ist-Position der Füße, die sich aus der Verbindungslinie zwischen Fersenmitte und zweitem Strahl der Fußzehen ergibt. Die Anlage zeigt in Echtzeit die Soll-Position der Füße in Form einer Projektion auf die Standplatte 1203, welche eine transparente (Kraftmess)-Platte sein kann. Die Soll-Position definiert sich durch biomechanische Regeln in einem Modell kombiniert mit statistischen Kennzahlen der weltbesten Golfspieler. Der Golfer bestimmt das Modell, nach welchem das Echtzeit-Feedback die Rückkopplung der Soll-Istwert Differenz an den Athleten rückmeldet.

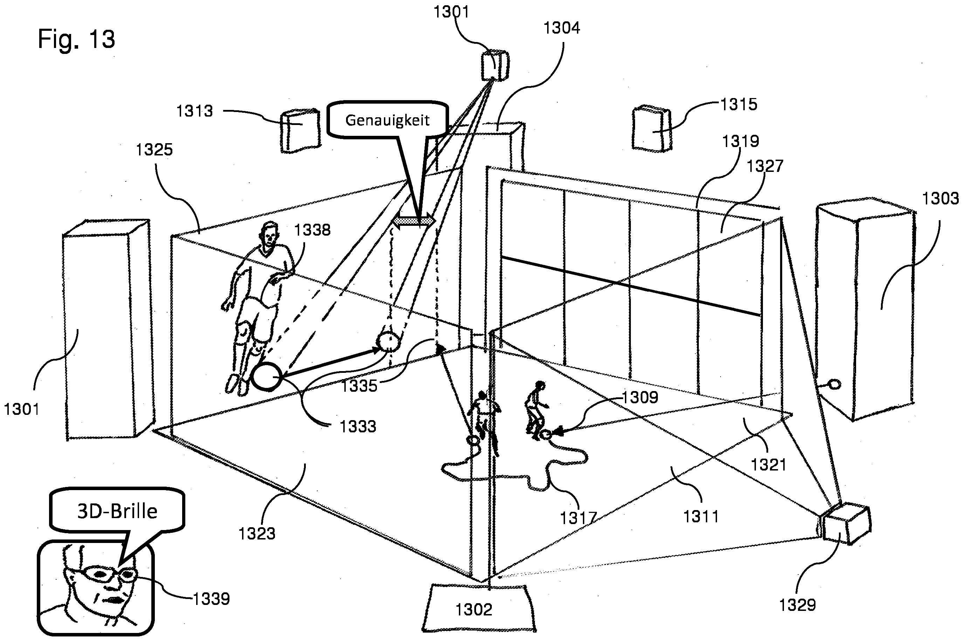

Fig. 13 zeigt ein System gemäß einer Ausführungsform, bei dem eine Bewegungsübung mit einem Ball, beispielsweise mit einem Fußball, unterstützt wird.

Das System umfasst zumindest eine Anzeigeeinrichtung 1301 und einen oder mehrere Ballgeber 1301, 1302,1303 und 1304, welche jeweils ausgebildet sind, einen Ball 1309 zum Spielfeld 1311 hin auszugeben. Das System umfasst zumindest eine Bestimmungseinrichtung 1313, 1315, welche ausgebildet ist, eine Position des Balls 1309 als Ist-Position des Balls zu erfassen. Hierzu kann der Ball 1309 ausgebildet sein, ein Sendesignal auszusenden, das die zumindest eine Bestimmungseinrichtung 1313, 1315 zur Positionsbestimmung empfangen und in an sich bekannter Weise auswerten kann. Die zumindest eine Bestimmungseinrichtung 1313, 1315 kann insbesondere ausgebildet sein, eine Mehrzahl von Positionen des Balls 1309 zu erfassen, welche in Fig. 13 durch die Trajektorie 1317 beispielhaft dargestellt ist. Auf diese Weise kann zumindest eine Bestimmungseinrichtung 1313, 1315 eine Verweildauer des Balls 1309 bei einem Spieler, welcher den Ball 1309 annimmt und abgibt, erfassen. Die Verweildauer kann beispielsweise auf der Basis einer Abbremsung, d.h. einer negativen Beschleunigung, des Balls 1309 zum Zeitpunkt der Ballannahme durch den Spieler sowie einer positiven Beschleunigung zum Zeitpunkt der Ballabgabe durch den Spieler erfasst werden.

Das System umfasst ferner zumindest einen Ballfänger 1319, 1321, 1323, 1325, welcher beispielsweise als eine Sensorwand ausgebildet ist, die das Spielfeld 1311 zumindest teilweise begrenzt. Die Sensorwände 1319, 1321, 1323 und 1325 umfassen beispielsweise Sensorbereiche 1327, welche mit Drucksensoren ausgestattet sein können. Hierzu kann eine Anzeigeeinrichtung 1329 beispielsweise einen Bereich 1327 anwählen, um eine Soll-Position des Balls 1309 anzuzeigen. Dies kann beispielsweise durch ein Anleuchten des jeweiligen Bereichs 1327 erfolgen. Auf diese Weise kann dem Spieler angezeigt werden, wohin er den Ball 1309 schießen soll.

Gemäß einer zusätzlichen oder alternativen Ausführungsform ist die Anzeigeeinrichtung 1301 vorgesehen, welche ausgebildet ist, eine Soll-Position 1333 des Balls auf eine der Sensorwände 1325 statisch oder dynamisch zu projizieren. Die Soll-Position kann beispielsweise, wie es in Fig. 13 dargestellt ist, veränderlich sein, was durch den Pfeil dargestellt ist. Die Sensorwand 1325 kann nach der Ballabgabe einen Einschlagsort 1335 erfassen, beispielsweise drucktechnisch, woraus eine Abweichung 1337 zwischen der Soll-Position und der erfassten Position bestimmt werden kann. Der Einschlagsort 1335 kann bei einer Lokalisation des Balles und einer dem Computer bekannten Raumgeometrie auch ohne Drucksensorik ermittelt werden. In diesem Fall kann auf die Drucksensorik bei der Sensorwand verzichtet werden.

Gemäß einer Ausführungsform kann die Anzeigeeinrichtung 1329 eine Spielfigur 1338 auf die Sensorwand 1325 projizieren, um einen Mitspieler zu simulieren. Diese Simulation kann dreidimensional sein, wozu der Spieler beispielsweise mit dreidimensionalen Gläsern 1339 ausgestattet werden kann, beispielsweise einer 3D-Brille.

Ein Vorteil des hierin beschriebenen Konzeptes besteht darin, dass es auf mehrere Ballsportarten übertragbar ist, aber auch in anderen Sportarten (Klettern) oder außersportlichen Bereichen, in denen motorisches Lernen bedeutsam ist oder motorisches Lernen eine Rolle spielt, einsetzbar ist. Weiterhin kann die gleiche Systematik zum Beispiel auf kleinere räumliche Anordnungen runtergebrochen werden und solche Systeme zum Bau neuer Geräte für Schausteller auf Jahrmärkten eingesetzt werden. Ebenso kann das System in Freizeitparks zum Bau neuer Attraktionen eingesetzt werden. Des Weiteren kann das System in der Rehabilitation von Schlaganfallpatienten oder anderen Menschen, die Bewegungen neu lernen müssen, eingesetzt werden, da das permanente Echtzeitfeedback die Lernzeiten im Vergleich zu Trial-And-Error-Lernen durch Versuch und Irrtum stark abkürzt. Da mit dieser Technologie Leistungsprofile von Profifußballern/anderen Sportlern hinsichtlich des technisch/taktischen Fähig- und Fertigkeitsniveaus erstellt werden können, ist damit zu rechnen, dass Profivereine Ihre Spieler vor einem Kauf oder Verkauf umfassend in der Anlage diagnostizieren lassen. Auf Grund der horrenden Summen für Verkäufe ist mit entsprechend hohen Beträgen für ein diagnostisches Dienstleistungsangebot im dargestellten Sinne zu erwarten.

Gemäß einer Ausführungsform ist ein Echtzeitlokalisationssystem von einem oder mehreren aktiven oder passiven Markern vorgesehen, das befestigt an einer oder mehreren Stellen eines oder mehrerer Subjekte und/oder eines oder mehrerer Objekte sein kann. Das Echtzeitlokalisationssystem umfasst beispielsweise zumindest eine Anzeigevorrichtung. Das Echtzeitlokalisationssystem kann ein Infrarotkinematographiesystem sein, das mit passiven retroreflexiven Markern funktioniert, es kann aber auch ein videobasiertes Trackingsystem, oder ein funkbasiertes Lokalisationssystem sein, dass mit aktiven Sendern und entsprechender Empfängertechnologie arbeitet. Es kann aber auch jedes andere aktive oder passive Lokalisationssystem sein.

Ferner kann ein Echtzeitbiosignalaufnahme- und Weiterleitungssystem zur synchronen Ableitung und Weiterleitung physiologischer Signale, z.B. Herzfrequenz, Herzfrequenzvariabilität, Köpertemperatur, Atemfrequenz, Hautleitwiederstand, Elektrolytzusammensetzung mit den vorstehend genannten Lokalisationsdaten vorgesehen sein. Darüber hinaus können technische Daten wie Luftdruck und/oder Rotation und/oder Beschleunigung berücksichtigt werden.

Ferner kann ein Datenbanksystem vorgesehen sein, das mit externen und/oder internen Daten gefüllt werden kann. Diese Datenbank wird permanent erweitert, entweder mit externen Daten oder internen Daten. Externe Daten sind zum Beispiel Daten aus Spielen oder Trainingseinheiten oder sonstigen Events oder Ereignissen, die per Video oder einer anderen Datenerfassungstechnik aufgezeichnet und analysiert worden sind. Die Datenbank nimmt auch Werte aus anderen Quellen auf. Zum Beispiel leistungsdiagnostische Kenngrößen aus Labor- und oder Feldstufentests oder auch anthropometrische Daten aus der Vermessung mit einem Bodyscanner, etc. Die Datenbank wird jedoch auch permanent und systematisch erweitert durch Daten, die aus den eigenen Aufnahmen (Istwerte) erfolgen. Diese Daten werden als interne Quellen bezeichnet.

Ferner kann ein Regelsystem vorgesehen sein, welches durch die Implementierung von Expertenwissen ständig erweitert wird. Experten können Sportwissenschaftler, Sportmediziner, Ingenieure, Fußballtrainer, etc. sein.

Ferner kann ein Setupmodul vorgesehen sein, das durch einen PC oder Tablet-PC oder Smartphone oder ein anderes Steuerungsgerät gesteuert wird. Dieses Setupmodul bestimmt, welche internen und externen Quellen für eine Trainings- Spiel oder Beobachtungseinheit zum Soll-Istwert Vergleich herangezogen werden. Ebenso wird über das Modul die Spezifikation für ein Echtzeitfeedbacksystem festgelegt.

Das Setupmodul, das Regelsystem und das Datenbanksystem können in der Bestimmungseinrichtung implementiert sein.

Ferner kann ein Echtzeitfeedbacksystem vorgesehen sein, das die Anzeigeeinrichtung umfasst und das Sollwerte und/oder Istwerte und/oder Soll-/Istwert-Differenzen in Echtzeit an das Subjekt, bzw. die Subjekte in Form von optisch/visuellen und/oder akustischen und/oder taktilen Signalen sendet. Visuelle Signale können sein: ein Laserprojektionssystem oder ein anderes optisches Projektionssystem oder ein Bildschirm oder ein anderes optische/visuelles Darstellungsverfahren, ein akustisches System, umfassend einen Miniaturkopfhörer oder eine Beschallungsanlage oder ein am Körper befindliches Lautsprechersystem, oder zumindest ein Vibrationsgerät, das am Körper an definierten Stellen getragen werden kann.

## Patentansprüche

1. System zur Unterstützung einer Bewegungsübung mit einem Objekt, wobei das Objekt ein Ball oder ein Puck ist, mit:
einer Erfassungseinrichtung (101) zum Erfassen einer Ist-Position des Objektes, wobei die Erfassungseinrichtung (101) ausgebildet ist, eine Mehrzahl von weiteren Ist-Positionen von einer Mehrzahl von weiteren Objekten zu bestimmen, wobei die Erfassungseinrichtung (101) als ein funkbasiertes Lokalisationssystem ausgebildet ist, wobei die Erfassungseinrichtung (101) einen Empfänger umfasst, und wobei das Objekt und jedes weitere Objekt einen jeweiligen Sender umfasst;
einer Bestimmungseinrichtung (103) zum Bestimmen einer Soll-Position des Objektes; und
einer Anzeigeeinrichtung (105) zum Anzeigen eines Hinweises auf die Soll-Position, wenn sich die Ist-Position von der Soll-Position unterscheidet,
wobei die Bestimmungseinrichtung (103) ausgebildet ist, einen Schwerpunkt der Mehrzahl von weiteren Ist-Positionen zu bestimmen, und die Soll-Position in Abhängigkeit von der Ist-Position des Objektes und dem Schwerpunkt, insbesondere relativ zu dem Schwerpunkt, zu bestimmen.

2. System nach Anspruch 1, wobei das Objekt ausgebildet ist, ein Positionssignal auszusenden, und wobei die Erfassungseinrichtung ausgebildet ist, das Positionssignal zu empfangen und die Ist-Position des Objektes auf der Basis des empfangenen Positionssignals zu bestimmen.

3. System nach einem der vorstehenden Ansprüche, wobei die Bestimmungseinrichtung ausgebildet ist, die Soll-Position in Abhängigkeit von der Ist-Position des Objektes und einer geometrischen Charakteristik eines Bereichs, insbesondere eines Spielfeldes, innerhalb dessen das Objekt bewegbar ist, oder die Soll-Position in Abhängigkeit von der Ist-Position des Objektes bezüglich einer geometrischen Charakteristik des Spielfeldes, insbesondere einer Tormitte, zu bestimmen.

4. System nach einem der vorstehenden Ansprüche, wobei die Anzeigeeinrichtung (105) ausgebildet ist, als Hinweis auf die Soll-Position die Soll-Position selbst oder einen Hinweis auf die Lage der Soll-Position bezüglich einer Lage des Objektes, insbesondere auf eine Richtung zu der Soll-Position, oder als einen Hinweis auf eine Differenz zwischen der Ist-Position und der Soll-Position anzuzeigen.

5. System nach einem der vorstehenden Ansprüche, wobei die Anzeigeeinrichtung (105) eine Sensorwand zum Detektieren eines auf die Sensorwand eintreffenden Objektes umfasst, und wobei die Anzeigeeinrichtung ausgebildet ist, einen Bereich (1333, 1327) der Sensorwand als Hinweis auf die Soll-Position durch visuelles Hervorheben, insbesondere durch Leuchten oder Beleuchten des Bereichs, oder durch akustisches Hervorheben anzuzeigen.

6. System nach einem der vorstehenden Ansprüche, wobei die Bestimmungseinrichtung (103) ausgebildet ist, die Soll-Position in Abhängigkeit von einem Körperparameter, insbesondere Herzfrequenz, Herzfrequenzvariabilität, Körpertemperatur, Blutwertparameter wie Zuckerkonzentration oder Sauerstoffkonzentration oder Atemfrequenz oder Schrittfrequenz oder Laufgeschwindigkeit zu bestimmen.

7. System nach einem der vorstehenden Ansprüche, das ferner einen Objektgeber, insbesondere einen Ballgeber oder einen Puckgeber, zum Ausgeben des Objektes umfasst.

8. Verfahren zur Unterstützung einer Bewegungsübung mit einem Objekt unter Verwendung eines Systems, wobei das System eine Erfassungseinrichtung (101), eine Bestimmungseinrichtung (103) und eine Anzeigeeinrichtung (105) umfasst, wobei die Erfassungseinrichtung (101) als ein funkbasiertes Lokalisationssystem ausgebildet ist, wobei die Erfassungseinrichtung (101) einen Empfänger umfasst, wobei das Objekt und jedes weitere Objekt einen jeweiligen Sender umfasst, wobei das Objekt ein Ball oder ein Puck ist, mit:
Erfassen einer Ist-Position des Objektes durch die Erfassungseinrichtung (101);
Bestimmen einer Mehrzahl von weiteren Ist-Positionen von einer Mehrzahl von weiteren Objekten durch die Erfassungseinrichtung (101);
Bestimmen eines Schwerpunktes der Mehrzahl von weiteren Ist-Positionen durch die Bestimmungseinrichtung (103);
Bestimmen einer Soll-Position des Objektes durch die Bestimmungseinrichtung (103); und
Anzeigen eines Hinweises auf die Soll-Position durch die Anzeigeeinrichtung (105), wenn sich die Ist-Position von der Soll-Position unterscheidet,
wobei die Soll-Position in Abhängigkeit von der Ist-Position des Objektes und dem Schwerpunkt, insbesondere relativ zu dem Schwerpunkt, bestimmt wird.

## Claims

1. A system for supporting a movement exercise with an object, wherein the object is a ball or a puck, comprising:
a detection device (101) for detecting an actual position of the object, wherein the detection device (101) is configured to determine a plurality of further actual positions from a plurality of further objects, wherein the detection device (101) is designed as a radio-based localization system, wherein the detection device (101) comprises a receiver and wherein the object and each further object comprises a respective transmitter;
a determination device (103) for determining a set point position of the object; and
a display device (105) for displaying an indication on the set point position when the actual position differs from the set point position,
wherein the determination device (103) is configured to determine a center of gravity of a plurality of further actual positions and to determine the set point position depending on the actual position of the object and the center of gravity, in particular relative to the center of gravity.

2. The system of claim 1, wherein the object is configured to emit a position signal and wherein the detection device is configured to receive the position signal and to determine the actual position of the object based on the received position signal.

3. The System according to one of the preceding claims, wherein the determination device is configured to determine the set point position depending on the actual position of the object and a geometric characteristic of an area, in particular a playing field, within which the object can be moved or the set point position depending on the actual position of the object with respect to a geometric characteristic of the playing field, in particular a goal center.

4. The system according to one of the preceding claims, wherein the display device (105) is configured to display the set point position itself as an indication to the set point position or an indication of the location of the set point position with respect to the location of the object, in particular a direction of the object to the set point position, or as an indication to a difference between the actual position and the set point position.

5. The system according to one of the preceding claims, wherein the display device (105) comprises a sensor wall for detecting an object arriving on the sensor wall and wherein the display device is configured to indicate an area (1333, 1327) of the sensor wall as an indication to the set point position by visual highlighting, in particular visual highlighting by illuminating or illuminating the area, or by acoustic highlighting.

6. The System according to one of the preceding claims, wherein the determination device (103) is configured to determine the set point position depending on a body parameter, in particular hearth rate, heart rate variability, body temperature, blood value parameters such as sugar concentration or oxygen concentration, respiratory rate, step frequency or running speed.

7. The System according to one of the preceding claims, further comprising an object dispenser, in particular a ball dispenser or a puck dispenser, for dispensing the object.

8. A method for supporting a movement exercise with an object using a system, wherein the system comprises a detection device (101), a determination device (103) and a display device (105), wherein the detection device (101) is designed as a radio-based localization system, wherein the detection device (101) comprises a receiver, wherein the object and each further object comprises a respective transmitter, wherein the object is a ball or a puck, comprising:
Detecting an actual position of the object by the detection device (101);
Determining a plurality of further actual positions from a plurality of further objects by the detection device (101);
Determining a center of gravity of the plurality of further actual positions by the determination device (103);
Determining a set point position of the object by the determination device (103); and
Displaying an indication on the set point position by the display device (105) when the actual position differs from the set point position, wherein the set point position is determined depending on the actual position of the object and the center of gravity, in particular determined relative to the center of gravity.

## Revendications

1. Système d'assistance à un exercice de mouvement avec un objet, l'objet étant une balle ou un palet, comprenant :
un dispositif de détection (101) destiné à détecter une position réelle de l'objet, le dispositif de détection (101) étant configuré pour déterminer une pluralité de positions réelles supplémentaires d'une pluralité d'objets supplémentaires, le dispositif de détection (101) étant réalisé sous la forme d'un système de localisation radioélectrique, le dispositif de détection (101) comportant un récepteur et l'objet et chaque objet supplémentaire comportant un émetteur respectif ;
un dispositif de détermination (103) destiné à déterminer une position de consigne de l'objet ; et
un dispositif d'affichage (105) destiné à afficher une notification à propos de la position de consigne lorsque la position réelle est différente de la position de consigne,
le dispositif de détermination (103) étant configuré pour déterminer un barycentre de la pluralité de positions réelles supplémentaires et pour déterminer la position de consigne en fonction de la position réelle de l'objet et du barycentre, notamment par rapport au barycentre.

2. Système selon la revendication 1, l'objet étant configuré pour émettre un signal de position et le dispositif de détection étant configuré pour recevoir le signal de position et pour déterminer la position réelle de l'objet en fonction du signal de position reçu.

3. Système selon l'une des revendications précédentes, le dispositif de détermination étant configuré pour déterminer la position de consigne en fonction de la position réelle de l'objet et d'une caractéristique géométrique d'une zone, notamment d'un terrain de jeu, à l'intérieur duquel l'objet peut être déplacé, ou pour déterminer la position de consigne en fonction de la position réelle de l'objet en référence à une caractéristique géométrique du terrain de jeu, notamment d'un centre de but.

4. Système selon l'une des revendications précédentes, le dispositif d'affichage (105) étant configuré pour afficher comme notification à propos de la position de consigne la position de consigne elle-même ou une notification à propos de la situation de position de consigne en référence à une situation de l'objet, notamment à propos d'une direction vers la position de consigne, ou sous la forme d'une notification à propos d'une différence entre la position réelle et la position de consigne.

5. Système selon l'une des revendications précédentes, le dispositif d'affichage (105) comportant une paroi de capteurs destinée à détecter un objet arrivant sur la paroi de capteurs, et le dispositif d'affichage étant configuré pour afficher une zone (1333, 1327) de la paroi de capteurs en tant que notification à propos de la position de consigne par une mise en valeur visuelle, notamment par des lampes ou un éclairage de la zone, ou par une mise en valeur sonore.

6. Système selon l'une des revendications précédentes, le dispositif de détermination (103) étant configuré pour déterminer la position de consigne en fonction d'un paramètre du corps, notamment la fréquence cardiaque, la variabilité de la fréquence cardiaque, la température du corps, les paramètres de niveaux sanguins comme la concentration de sucre ou la concentration d'oxygène ou la fréquence respiratoire ou la fréquence des pas ou la vitesse de marche.

7. Système selon l'une des revendications précédentes, comportant en outre un transmetteur d'objet, notamment un transmetteur de balle ou un transmetteur de palet, destiné à délivrer l'objet.

8. Procédé d'assistance à un exercice de mouvement avec un objet en utilisant un système, le système comportant un dispositif de détection (101), un dispositif de détermination (103) et un dispositif d'affichage (105), le dispositif de détection (101) étant réalisé sous la forme d'un système de localisation radioélectrique, le dispositif de détection (101) comportant un récepteur et l'objet et chaque objet supplémentaire comportant un émetteur respectif, l'objet étant une balle ou un palet, comprenant :
détection d'une position réelle de l'objet par le dispositif de détection (101) ;
détermination d'une pluralité de positions réelles supplémentaires d'une pluralité d'objets supplémentaires par le dispositif de détection (101) ;
détermination d'un barycentre de la pluralité de positions réelles supplémentaires par le dispositif de détermination (103) ;
détermination d'une position de consigne de l'objet par le dispositif de détermination (103) ; et
affichage d'une notification à propos de la position de consigne par le dispositif d'affichage (105) lorsque la position réelle est différente de la position de consigne, la position de consigne étant déterminée en fonction de la position réelle de l'objet et du barycentre, notamment par rapport au barycentre.
